# EUROPEAN PATENT SPECIFICATION

(11) **EP 4 399 888 B1**
(45) Date of publication and mention of the grant of the patent: **03.06.2026**
(21) Application number: 22761499.7
(22) Date of filing: 04.08.2022
(51) Int. Cl.: H04W 4/38, H04W 4/029, H04W 24/10, H04W 4/70, H04W 24/04

(54) **INTERNET OF THINGS BASED AIR POLLUTION PREVENTION IN 5TH GENERATION NETWORKS**
INTERNET-DER-DINGE-BASIERTE LUFTVERSCHMUTZUNGSVERHINDERUNG IN NETZWERKEN DER 5. GENERATION
PRÉVENTION DE LA POLLUTION DE L'AIR BASÉE SUR L'INTERNET DES OBJETS DANS DES RÉSEAUX DE 5 GÉNÉRATION

(30) Priority: 09.09.2021 EP 21382808
(43) Date of publication of application: 17.07.2024
(73) Proprietor: Telefonaktiebolaget LM Ericsson (publ), 164 83 Stockholm (SE)
(72) Inventor: MUÑOZ DE LA TORRE ALONSO, Miguel Angel, 28002 Madrid (ES)
(74) Representative: Zacco Sweden AB
(86) International application number: PCT/EP2022/071927
(87) International publication number: WO 2023/036525

(56) References cited:
- WO-A1-2021/155090
- US-A1- 2020 196 169
- ERICSSON: "Data collection from AF support for internal group ID", vol. SA WG2, no. e-meeting; 20210816 - 20210827, 7 September 2021 (2021-09-07), XP052050003, Retrieved from the Internet <URL:https://ftp.3gpp.org/3guInternal/3GPP_Ultimate_CRPacks/SP-210921.zip 23288_CR0389r1_(Rel-17)_S2-2106607_was05443r01.docx> [retrieved on 20210907]
- ERICSSON ET AL: "KI#8 - Solution#27- UE data as an input for analytics generation", vol. SA WG2, no. Electronic; 20200819 - 20200901, 2 September 2020 (2020-09-02), XP051928786, Retrieved from the Internet <URL:https://ftp.3gpp.org/tsg_sa/WG2_Arch/TSGS2_140e_Electronic/Docs/S2-2006252.zip S2-2006252_5107r02_KI#8_ updates to solution#27.doc> [retrieved on 20200902]
- QUALCOMM INCORPORATED: "User Plane Based UE Data Collection", vol. SA WG2, no. Elbonia; 20200601 - 20200612, 22 May 2020 (2020-05-22), XP051889871, Retrieved from the Internet <URL:https://ftp.3gpp.org/tsg_sa/WG2_Arch/TSGS2_139e_Electronic/Docs/S2-2003863.zip S2-2003863_TR23700-91-Solution to KI#8_user plane based UE data collection _v3.doc> [retrieved on 20200522]

## Description

### TECHNICAL FIELD

The present disclosure relates generally to communications, and more particularly to communication methods and related devices and nodes supporting wireless communications.

### BACKGROUND

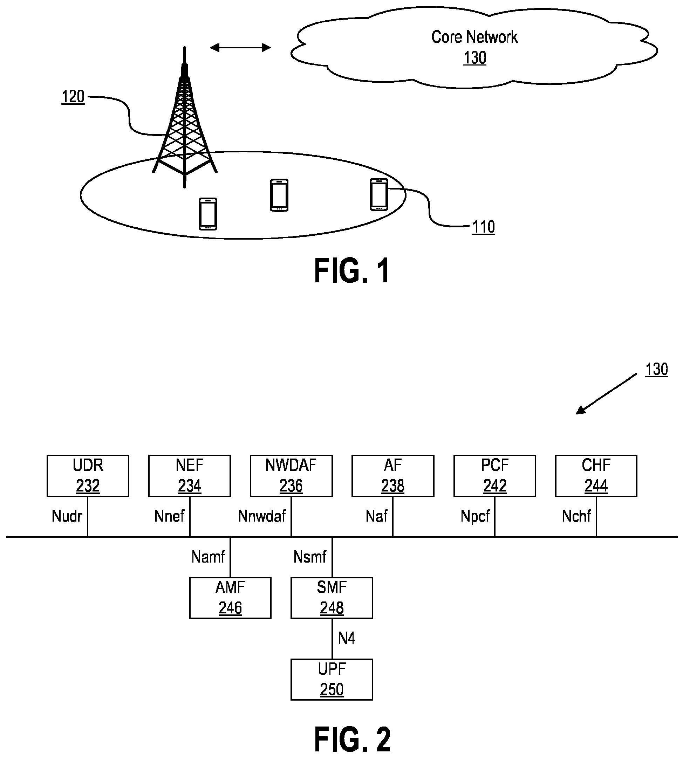

FIG. 1 illustrates an example of a new radio ("NR") network (e.g., a 5th Generation ("5G") network) including a 5G core ("5GC") network 130, network node 120 (e.g., a 5G base station ("gNB")), multiple communication devices 110 (also referred to as user equipment ("UE")).

FIG. 2 illustrates an example of a reference architecture of a 5GC network 130 as defined by the 3rd generation partnership project ("3GPP"). In this example, the 5GC network includes a unified data repository ("UDR") 232, a network exposure function ("NEF") 234, a network data analytics function ("NWDAF") 236, an application function ("AF") 238, a policy control function ("PCF") 242, a charging function ("CHF") 244, an access and mobility management function ("AMF") 246, and a session management function ("SMF") 248 all communicatively coupled to each other. The 5GC network further includes a user plane function ("UPF") 250 communicatively coupled to the SMF 248.

The NWDAF 236 represents an operator managed network analytics logical function. The NWDAF 236 is part of the 5GC architecture and uses the mechanisms and interfaces specified for 5GC and operations, administration, and maintenance ("OAM"). The NWDAF 236 interacts with different entities for different purposes including: data collection based on event subscription, provided by the AMF 246, the SMF 248, the PCF 242, a unified data management ("UDM"), the AF 238 (directly or via the NEF 234), and OAM; retrieval of information from data repositories (e.g., UDR 232 via UDM for subscriber-related information); retrieval of information about network functions ("NFs") (e.g., NRF for NF-related information, and network slice selection function ("NSSF") for slice-related information); and on demand provision of analytics to consumers.

The UDR 232 stores data grouped into distinct collections of subscription-related information: subscription data; policy data; structured data for exposure; and application data.

The PCF 242 supports a unified policy framework to govern the network behavior. Specifically, the PCF 242 provides policy and charging control ("PCC") rules to the policy and charging enforcement function ("PCEF") (e.g., the SMF/UPF that enforces policy and charging decisions according to provisioned PCC rules).

The AMF 246 manages UE access (e.g., when UE is connected through different access networks) and UE mobility aspects.

The SMF 248 supports different functionalities (e.g., SMF 248 receives PCC rules from the PCF 242 and configures the UPF 250 accordingly).

The UPF 250 supports handling of user plane traffic based on the rules received from the SMF 248 (e.g., packet inspection and different enforcement actions such as quality of service ("QoS") handling).

Air pollution in cities is a serious environmental problem, especially in developing countries. The air pollution path of an urban atmosphere includes emission and transmission of air pollutants resulting in ambient air pollution. Each part of the path is influenced by different factors. In some examples, emissions from motor traffic are a significant source group throughout the world. During transmission, air pollutants are dispersed, diluted, and subjected to photochemical reactions.

Some of the major pollutants from motor vehicles include: particulate matter ("PM"); volatile organic compounds ("VOCs"); nitrogen oxide ("NOx"); carbon monoxide ("CO"); sulfur dioxide ("SO2"); and other greenhouse gasses.

One type of particulate matter is the soot seen in vehicle exhaust. Fine particles (e.g., less than one-tenth the diameter of a human hair) pose a serious threat to human health, as they can penetrate deep into the lungs. PM can be a primary pollutant or a secondary pollutant from hydrocarbons, nitrogen oxides, and sulfur dioxides. In some examples, diesel exhaust is a major contributor to PM pollution.

These pollutants react with nitrogen oxides in the presence of sunlight to form ground level ozone, a main ingredient in smog. Though beneficial in the upper atmosphere, at the ground level this gas irritates the respiratory system, causing coughing, choking, and reduced lung capacity. VOCs emitted from cars, trucks and buses, which include the toxic air pollutants benzene, acetaldehyde, and 1,3-butadiene, are linked to different types of cancer.

NOx pollutants form ground level ozone and particulate matter (secondary). Also harmful as a primary pollutant, NOx can cause lung irritation and weaken the body's defenses against respiratory infections such as pneumonia and influenza.

CO is an odorless, colorless, and poisonous gas formed by the combustion of fossil fuels such as gasoline and is emitted primarily from cars and trucks. When inhaled, CO blocks oxygen from the brain, heart, and other vital organs.

Power plants and motor vehicles create SO2 by burning sulfur-containing fuels, especially diesel and coal. Sulfur dioxide can react in the atmosphere to form fine particles and, as other air pollutants, poses the largest health risk to young children and asthmatics.

Motor vehicles also emit greenhouse gasses, predominantly carbon dioxide, that contribute to global climate change. In fact, tailpipe emissions from cars, trucks and buses account for over one-fifth of the United States' total global warming pollution; transportation, which includes and airplanes, trains, and ships accounts for around thirty percent of all heat-trapping gas emissions.

The publication "Data collection from AF support for internal group ID" discloses 3GPP specification changes to clarify and enhance how a Network Data Analytics Function (NWDAF) collects data from an Application Function (AF) using internal or external group identifiers.

The publication "KI#8-Solution#27 - UE data as an input for analytics generation" discloses a study on enhancing the 5G system for the NWDAF to collect and utilize data provided by the UE for analytics, exploring methods such as using an MNO-controlled Application Function (AF) to gather data from the UE application client over the user plane.

The patent application WO 2021/155090 A1 discloses methods, systems, and devices may assist in enhancements to PMF protocol and new ATSSS steering modes to enable support for analytics driven or multi-USIM steering.

The patent application US 2020/196169 A1 discloses a network data analytics (NWDA) function and method of policy optimization in a communication network .

The publication "User Plane Based UE Data Collection" discloses a method for an Application Function (AF) to collect application-specific data from a UE by having the UE send the data in a transparent container over the user plane through the NWDAF to the AF.

### SUMMARY

The invention is defined by the independent claims. Furthermore, the embodiments of the invention are those defined by the claims. Moreover, examples and embodiments, which are not covered by the claims, are presented not as embodiments of the invention, but as background art or examples useful for understanding the invention. According to some embodiments, a method performed by a network node for determining analytics associated with a communication device is provided. The method includes determining an identifier associated with a communication device using a characteristic of the communication device. The method further includes transmitting a message requesting that the communication device gather and report data. The method further includes, responsive to transmitting the message, receiving the data. The method further includes generating the analytics based on the data.

Additional embodiments herein describe network nodes, computer programs, and computer program products for performing the operations in the above method embodiments.

Various embodiments described herein allow a network operator to get real-time data and analytics related to vehicle pollution and permit governments and local administrations to make more effective decisions to reduce air pollution.

### BRIEF DESCRIPTION OF THE DRAWINGS

The accompanying drawings, which are included to provide a further understanding of the disclosure and are incorporated in and constitute a part of this application, illustrate certain non-limiting embodiments of inventive concepts. In the drawings:
FIG. 1 is a schematic diagram illustrating an example of a 5^{th} generation ("5G") network;
FIG. 2 is a block diagram illustrating an example of a 5G network architecture;
FIG. 3 is a schematic diagram illustrating an example of a 5G network for preventing air pollution according to some embodiments of inventive concepts;
FIG. 4 is a block diagram illustrating an example of a 5G network for preventing air pollution according to some embodiments of inventive concepts;
FIG. 5 is a signal flow diagram illustrating an example of operations performed by a network data analytics function ("NWDAF") node for preventing air pollution according to some embodiments of inventive concepts;
FIG. 6 is a signal flow diagram illustrating an example of operations performed by an internet of things ("IoT") analytics entity for preventing air pollution according to some embodiments of inventive concepts;
FIG. 7 is a block diagram illustrating an example of a communication device (sometimes referred to herein as a user equipment ("UE")) according to some embodiments of inventive concepts;
FIG. 8 is a block diagram illustrating an example of a radio access network ("RAN") node (e.g., a base station eNB/gNB) according to some embodiments of inventive concepts;
FIG. 9 is a block diagram illustrating an example of a core network ("CN") node (e.g., an access mobility management function ("AMF") node and a session management function ("SMF") node) according to some embodiments of inventive concepts;
FIG. 10 is a flow chart illustrating operations of a network node according to some embodiments of inventive concepts;
FIG. 11 is a block diagram of a communication system in accordance with some embodiments;
FIG. 12 is a block diagram of a user equipment in accordance with some embodiments
FIG. 13 is a block diagram of a network node in accordance with some embodiments;
FIG. 14 is a block diagram of a host computer communicating with a user equipment in accordance with some embodiments;
FIG. 15 is a block diagram of a virtualization environment in accordance with some embodiments; and
FIG. 16 is a block diagram of a host computer communicating via a base station with a user equipment over a partially wireless connection in accordance with some embodiments in accordance with some embodiments.

### DETAILED DESCRIPTION

Inventive concepts will now be described more fully hereinafter with reference to the accompanying drawings, in which examples of embodiments of inventive concepts are shown. Inventive concepts may, however, be embodied in many different forms and should not be construed as limited to the embodiments set forth herein. Rather, these embodiments are provided so that this disclosure will be thorough and complete, and will fully convey the scope of present inventive concepts to those skilled in the art. It should also be noted that these embodiments are not mutually exclusive. Components from one embodiment may be tacitly assumed to be present/used in another embodiment.

There currently exist certain challenge(s). For example, air pollution in cities is a serious environmental problem and the biggest contributing factor is related to the vehicles' emissions. Governments and local administrations are introducing driving restrictions in large cities. However, air pollution has a big temporal and spatial variability and the current measurements are mostly based on local meteorological stations.

Certain aspects of the disclosure and their embodiments may provide solutions to these or other challenges.

Various embodiments described herein propose a mechanism which solves the above problems and is based on an internet of things ("IoT") cloud platform (such as the Ericsson IoT platform), hosting an IoT Analytics entity running analytics based on the collected data from one or more of: IoT devices; connected vehicles; 5GC entities; and external entities.

IoT devices include connected vehicles and connected meteorological stations. connected vehicles can provide data, for example, global positioning system ("GPS") position, vehicle speed, and engine status (e.g., on or off). Connected meteorological stations can provide data, for example, geographical coordinates, air pollution data like levels of PM, VOC, NOx, CO, SO2, and other greenhouse gases.

5GC entities (e.g., an AMF, a UDM/UDR, a NWDAF, and a NEF) can provide network related parameters and analytics.

External entities (e.g., institutions) can provide weather measurements and/or forecasts.

In some embodiments, a type of analytic relative to vehicle pollution is proposed, which allows an external entity (e.g., a government or local administration) to obtain useful data to plan and prevent air pollution in cities.

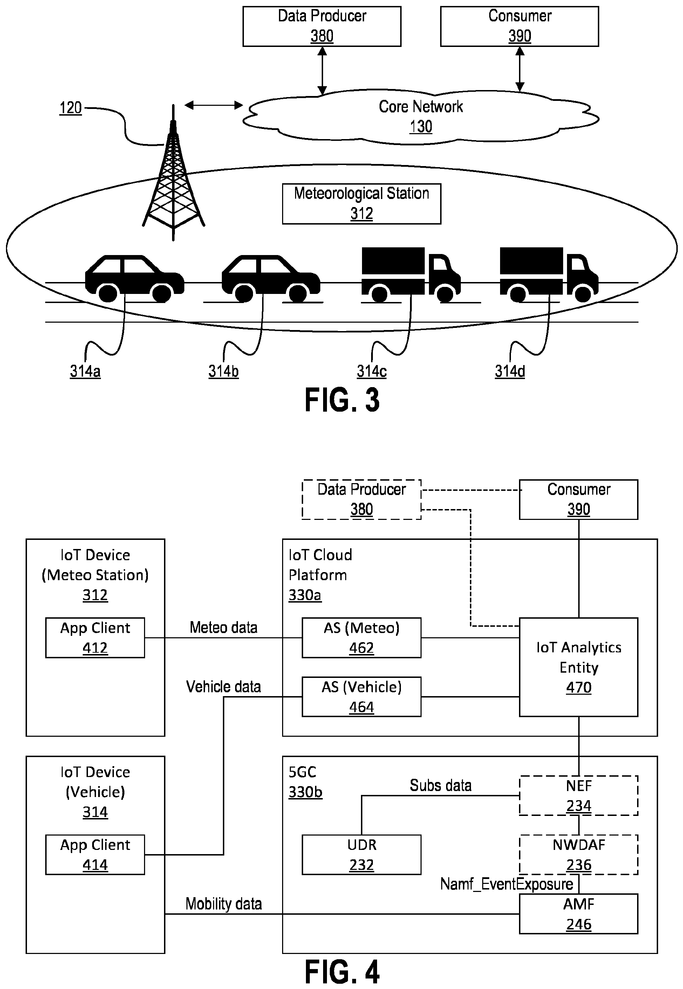

FIGS. 3-4 illustrate examples of a 5G network architecture for air pollution prevention. FIG. 3 illustrates a 5G network including a network node 120 coupled to a core network 130, which is coupled to a data producer 380 and a consumer 390. A roadway having IoT devices (vehicles) 314 (e.g., cars 314a-b and trucks 314c-d) and an IoT device (meteorological station) 312 can be within a coverage area of the network node 120. In some examples, the vehicles 314 can each provide information (e.g., vehicle speed) to the core network 130 via the network node 120. In additional or alternative examples, the meteorological station 312 can measure vehicle pollutants and provide the data to the core network 130 via the network node 120. In additional or alternative examples, the data producer 380 (e.g., a weather forecaster) can provide additional data (e.g., a weather forecast) to the core network 130. The core network 130 can perform data analytics on the collected data and provide a recommendation for how to handle air pollution to the consumer 390.

FIG. 4 is a block diagram illustrating an example of a 5G network for analyzing or preventing air pollution according to some embodiments. The 5G network can include a pair of IoT devices 312, 314 associated with a meteorological station and a vehicle, respectively. The 5G network can further include a IoT Clout platform 330a, a 5G core network 330b, a data producer 380, and a consumer 390. The IoT devices 312, 314 can each include an application client 412, 414 communicatively coupled to respective application servers 462, 464 in the IoT Cloud platform 330a that are each able to communicate data from the IoT devices 312, 314 to the IoT Analytics Entity 470. Thereby, IoT devices 312, 314 are examples for communication devices as described herein. The IoT Analytics Entity 470 can also be coupled to a NEF 234, NWDAF 236, and/or AMF 246 in the 5GC 330b, which can be coupled to the vehicle 314 for communicating mobility data from the vehicle 314 to the IoT Analytics Entity 470. The IoT Analytics Entity 470 can also be communicatively coupled to a UDR 232 of the 5GC, which can store identifiers associated with the different IoT devices 312, 314.

In some embodiments, as part of the subscriber data, IoT device subscription parameters are stored in the UDR 232. In some examples, the IoT device subscriptions are for vehicles and include a parameter indicating if the IoT device is a vehicle, e.g. for the exemplary IoT device 314. If the IoT device is a vehicle, or is associated with a vehicle, it can include parameters including: VehicleType (e.g., Car, Truck, or Bus); VehicleBrand (e.g., Volvo or Toyota); VehicleFuelType (e.g., Diesel, Biodiesel, Gasoline, Hybrid, or Electric); VehicleManufacturingYear (e.g., 2002).

In additional or alternative examples, the IoT device subscriptions are for meteorological stations and include a parameter indicating if the IoT device is a meteorological station (with the capability of measuring vehicle pollutants), e.g. for the exemplary IoT device 312. If the IoT device is a meteorological station, the IoT device subscription can include a parameter indicating a location of the IoT device.

A consumer 390 (e.g., an AF as a government or a local administration) can subscribe to an IoT Analytics entity 470 related to a new analytic (Analytic-ID=VehiclePollution), by triggering a AnalyticsSubscription_Subscribe Request message including the following parameters. In some examples, the parameters include an Analytic-ID=VehiclePollution. In additional or alternative examples, the parameters include an Analytic-Type=Statistical/Predictive/Both. It requests vehicle pollution analytics based on the analysis of existing data (Statistical) and/or the request is about predictions about the vehicle pollution in the future, based on the analysis of existing data and projections into a target date (Predictive). In additional or alternative examples, the parameters include a list of locations (e.g. a single location like a city: Madrid; or a list of locations within a city: Madrid and the different districts within Madrid: Latina, Moratalaz, Centro, etc). The latter requests to calculate the Analytic-ID for each location (e.g. district). In additional or alternative examples, the parameters include Time-Parameters (e.g., start analytic immediately and report the analytic result periodically on a per daily basis). In additional or alternative examples, the parameters include a list of UE (UE-ID e.g., to track a certain vehicle, UE-Group-ID, e.g. devices from a certain IoT provider). In additional or alternative examples, the parameters include a VehicleType (eg., Any, Car, Truck, Bus, etc). In additional or alternative examples, the parameters include a VehicleBrand (e.g., Any, Volvo, Toyota). In additional or alternative examples, the parameters include a VehicleFuelType (e.g., Any, Diesel, Biodiesel, Gasoline, Hybrid, Electric). In additional or alternative examples, the parameters include a VehicleManufacturingYearPeriod (e.g., before 2005). In additional or alternative examples, the parameters include driving restrictions parameters (e.g., on a certain date, there will be a driving restriction for vehicles of certain type).

As an example, an AF (for example associated with local administration) might subscribe to bus pollution for each of the different districts of a certain city, e.g. Madrid.

Based on the above analytic subscription, the IoT Analytics entity 470 can trigger data collection as follows. In some examples, the IoT Analytics entity 470 can trigger data collection from UDR/UDM, to retrieve the following data: a list of vehicles matching the requested conditions (VehicleType, VehicleBrand, VehicleFuelType, VehicleManufacturingYearPeriod and inside the requested location); and a list of meteorological stations inside the requested location (e.g., Madrid).

Based on the above, the IoT Analytics entity 470 can discover any active PDU sessions for the vehicles matching the above conditions (VehicleType, VehicleBrand, VehicleFuelType, VehicleManufacturingYearPeriod and inside the requested location). It is assumed the meteorological stations have always on (active) PDU sessions (in case this is not true, their PDU sessions will also need to be discovered).

In additional or alternative examples, the IoT Analytics entity 470 can trigger data collection from an AMF, to retrieve the following data for the vehicles matching the above conditions (and passing as input the requested location to filter out vehicles outside the requested location): Mobility and trajectory.

In additional or alternative examples, the IoT Analytics entity 470 can trigger data collection from the IoT (communication) device (either from an application client or from an application server) to retrieve the following data. From the vehicles matching the above conditions (e.g. VehicleType, VehicleBrand, VehicleFuelType, VehicleManufacturingYearPeriod, within the requested location and with active PDU sessions), vehicle statistics like GPS positions and timestamps, Vehicle speed, and Engine ON/OFF can be retrieved. From the meteorological stations inside the requested location, Meteorological station data (e.g., Geographical coordinates, air pollution data like levels of PM (Particulate matter), VOC (Volatile Organic Compounds), NOx (Nitrogen oxides), CO (Carbon monoxide), SO2 (Sulfur dioxide), Greenhouse gases) can be retrieved.

In additional or alternative examples, the IoT Analytics entity 470 can trigger data collection from external entities (e.g., to collect the weather forecast) in order to improve analytics result and optionally send a recommendation (e.g., if the weather forecast for the following days is rainy, the vehicle restrictions to be planned for those days might be relaxed).

Based on the data collected above, the IoT Analytics entity runs analytic processes (e.g., by using Machine Learning, by identifying patterns or behaviors among the vehicles of certain characteristics and/or by correlating data from the vehicles and the meteorological stations, etc) and generates the Analytic-Result, including the following information: Analytic-ID= VehiclePollution; PollutionMap (which includes at each geographical coordinate, an intensity level (e.g. from white to black in grey scales) which will be based on 1) number of target vehicles passing through that geographical coordinate, 2) time spent by target vehicles at that geographical coordinate (e.g., vehicle stopped due to traffic light or traffic jam), having into account vehicle data like Engine ON/OFF (e.g. in case a vehicle turns off the engine while stopped at a traffic light or traffic jam), and 3) vehicle speed when passing through that geographical coordinate (as emissions depend on the vehicle speed); prediction on vehicle pollution (e.g., based on historical data from previous analytic results) showing the future projection; List of locations with higher vehicle pollution; List of vehicles (UE-IDs) or vehicle types which contribute the most to the Vehicle pollution; statistics (on a per time period, e.g., weekdays vs weekend) on percentage of vehicles of a certain type (e.g. Car, Truck, Bus), brand (e.g., Volvo, Toyota), fuel type (Diesel, Biodiesel, Gasoline, Hybrid, Electric), manufacturing year (e.g., before 2005), etc; and recommendation actions (e.g., possible vehicle restrictions to apply by the consumer).

Based on the Analytic-Result, the Consumer (e.g., AF as a government or local administration) applies the corresponding actions (e.g., planning relative to vehicle restrictions on a per district basis). Such actions may relate to imposing restrictions to pollution sources, for example to restrict access to certain areas or particular streets for vehicles of a certain type (e.g. trucks, vehicles with Diesel engines, all motor vehicles except electric-driven vehicles, and/or vehicles assigned to a certain emission class), to impose speed limits in certain areas or streets, to certain types of vehicles or the like. This may also include restrictions to non-mobile pollution sources like power plants or manufacturing sites. Further, such actions may relate to reducing exposure of persons to polluting agents, like guiding pedestrian streams around areas of higher pollution, or perform protective measures on buildings, like closing of windows, activation of filter appliances or the like. The type and extent of actions can be based on parameters used or created in the analytic process, like type of pollutant(s), pollution map or location etc.

Various embodiments described herein propose a mechanism which allows the network operator to support government and administrations to prevent pollution in cities, based on IoT and Analytics in 5G networks.

Certain embodiments may provide one or more of the following technical advantage(s). The most relevant advantages of the proposed innovation include that some embodiments allow the network operator to get real time data and analytics related to vehicle pollution in cities, as a powerful tool allowing governments and local administrations to take the best decisions to fight against air pollution.

Various embodiments herein include determining an analytic relative to vehicle pollution, which allows an external entity acting as consumer (e.g., a government or a local administration) to obtain useful data to plan and prevent air pollution in cities.

In some embodiments, an IoT Analytics entity 470 (e.g., hosted in the IoT Cloud Platform 330a of FIG. 4) produces the analytic. In additional or alternative embodiments, a NWDAF produces the analytic.

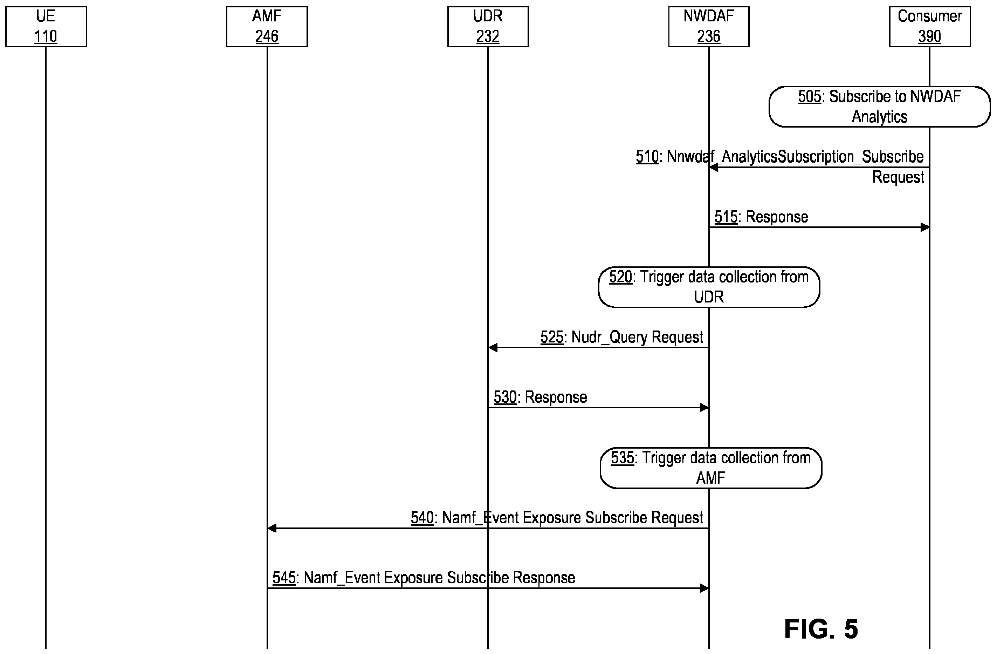

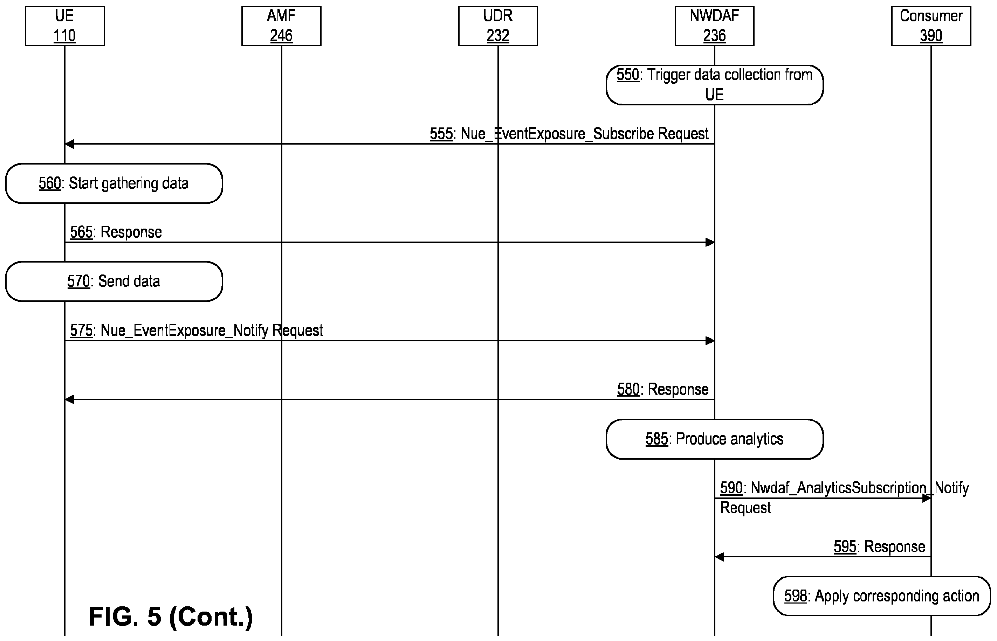

Not shown in the sequence diagram of FIG. 5, but it is also possible that the IoT Analytics entity 470 also triggers data collection from external entities (e.g. to collect the weather forecast) in order to improve analytics result and optionally send a recommendation (e.g. if the weather forecast for the following days is rainy, the vehicle restrictions to be planned for those days might be relaxed).

Finally, the analytic result (e.g., as a pollution heat map) is based on the UEs resp. communication devices camping on operator's mobile network (with respect to the UEs camping on other mobile networks in the same location, or with respect to UEs not connected to any mobile network, e.g. not IoT enabled). In any case, the data is assumed to be statistically relevant and it might be further sampled (i.e. the IoT Analytics entity does not need to collect data from all the mobile network connected vehicles in the target location).

Operations of a NWDAF 236 are illustrated in FIG. 5 and described below. In these embodiments, the NWDAF 236 is the entity producing the analytic. As a precondition, as part of the subscriber data, the following parameters are stored in the UDR 232: IoT device subscriptions (Vehicles); and IoT device subscriptions (Meteorological stations). The IoT device subscriptions can indicate whether the IoT device is a vehicle. If yes, the IoT device subscription may further indicate a VehicleType (e.g., Car, Truck, Bus, etc), a VehicleBrand (e.g., Volvo, Toyota), a VehicleFuelType (e.g., Diesel, Biodiesel, Gasoline, Hybrid, Electric), and a VehicleManufacturingYear (e.g., 2002). The IoT device subscriptions (can indicate whether the IoT is a meteorological station (with the capability of measuring vehicle pollutants). If yes, the IoT device subscription may further indicate a location of the meteorological station.

At block 505, consumer 390 (e.g., an AF associated with a government or a local administration) subscribes to the NWDAF 236 related to a new analytic (Analytic-ID=VehiclePollution), by, at block 510, transmitting a Nnwdaf_AnalyticsSubscription_Subscribe Request message including the following parameters. In some examples, the parameters include an Analytic-ID=VehiclePollution. In additional or alternative examples, the parameters include an Analytic-Type=Statistical/Predictive/Both. It requests vehicle pollution analytics based on the analysis of existing data (Statistical) and/or the request is about predictions about the vehicle pollution in the future, based on the analysis of existing data and projections into a target date (Predictive). In additional or alternative examples, the parameters include a list of locations (e.g. a single location like a city: Madrid; or a list of locations within a city: Madrid and the different districts within Madrid: Latina, Moratalaz, Centro, etc). The latter requests to calculate the Analytic-ID for each location (e.g. district). In additional or alternative examples, the parameters include Time-Parameters (e.g., start analytic immediately and report the analytic result periodically on a per daily basis). In additional or alternative examples, the parameters include a list of UE (UE-ID e.g., to track a certain vehicle, UE-Group-ID, e.g. devices from a certain IoT provider). In additional or alternative examples, the parameters include a VehicleType (eg., Any, Car, Truck, Bus, etc). In additional or alternative examples, the parameters include a VehicleBrand (e.g., Any, Volvo, Toyota). In additional or alternative examples, the parameters include a VehicleFuelType (e.g., Any, Diesel, Biodiesel, Gasoline, Hybrid, Electric). In additional or alternative examples, the parameters include a VehicleManufacturingYearPeriod (e.g., before 2005). In additional or alternative examples, the parameters include driving restrictions parameters (e.g., on a certain date, there will be a driving restriction for vehicles of certain type).

In the example shown in the sequence diagram of FIG. 5, the AF (local administration) can subscribe to truck pollution for the city of Madrid.

At block 515, the NWDAF 236 answers the request message (from block 510) with a successful response (accepting the request).

At block 520, the NWDAF 236 triggers data collection from the UDR 232/UDM. In the sequence diagram of FIG. 5, data collection from the 232 UDR is shown. In this example, the NWDAF 236 requests from the UDR 232 a list of UE-IDs for both the target vehicles and target meteorological stations (UE 110) in the target location.

Although not shown in the sequence diagram of FIG. 5, it is also possible to collect data from a UDM (which stores data for active PDU sessions).

At block 525, the NWDAF 236 transmits a Nudr_Query request message referring to the UE 110. In some examples, the Nudr_Query request message indicates a VehicleType=Truck, and Location=Madrid as input parameters. In other examples, the Nudr_Query request message indicates MeteoStations and Location=Madrid as input parameters.

At block 530, the UDR 232 finds the UE-IDs matching the input parameters in the request and transmits a Nudr_Query response including the list of UE-IDs. (e.g., Trucks in Madrid or MeteoStations in Madrid).

At blocks 535, for each of the UE-IDs in the list retrieved above, the NWDAF 236 discovers the AMF 246 serving the PDU session and triggers data collection from the AMF 236 (for the target vehicles and/or target meteorological station). In order to do this, at block 540, the NWDAF 236 transmits a Namf_Event Exposure Subscribe Request including as parameters: Event-ID=Mobility, Trajectory (e.g. as a single event for simplicity in this example, but in general these are two separate events); UE-ID; and Location=Madrid.

At block 545, if the UE-ID is in the target location, the AMF 246 tracks its mobility and trajectory, and reports it in a Namf_Event Exposure Notification Request including as parameters: Event-ID=Mobility,Trajectory; UE-ID; and Mobility Trajectorylnfo.

At blocks 550 and 555, for each of the UE-IDs (vehicles and/or meteorological stations) in the list retrieved above (the ones inside the target location), the NWDAF 236 triggers data collection from the UE 110. In order to do this for vehicle data, the NWDAF 236 triggers a Nue_EventExposure_Subscribe request message including the following parameters: Event-ID= VehicleData; UE-ID. In order to do this for meteorological station data, the NWDAF 236 triggers a Nue_EventExposure_Subscribe request message including the following parameters: Event-ID= MeteoStationData; and UE-ID.

At blocks 560 and 565, the UE 110 starts gathering data (e.g. a vehicle may gather data for Event-ID=VehicleData) and answers the request message in block 555 with a successful response (accepting the request).

At blocks 570 and 575, the UE 110 sends (e.g., periodically) data. In some examples, a vehicle may transmit data for Event-ID=VehicleData by triggering a Nue_EventExposure_Notify request including the following parameters: Event-ID= VehicleData; UE-ID; and VehicleData (e.g., GPS positions and timestamps, Vehicle speed, Engine ON/OFF, etc). In some examples, a Meteo Station sends (e.g. periodically) data for Event-ID=MeteoStationData by triggering a Nue_EventExposure_Notify request including the following parameters: Event-ID= MeteoStationData; UE-ID; and MeteostationData, e.g. Geographical coordinates, air pollution data like levels of PM (Particulate matter), VOC (Volatile Organic Compounds), NOx (Nitrogen oxides), CO (Carbon monoxide), SO2 (Sulfur dioxide), Greenhouse gases, etc.

At block 585, the NWDAF 236 produces analytics based on the data collected from the UDR 232/UDM, the AMF 246, and the UE 110. Specifically, the NWDAF 236 runs analytic processes (e.g., by using Machine Learning, by identifying patterns or behaviors among the vehicles of certain characteristics and/or by correlating data from the vehicles and the meteorological stations, etc) and generates the information. The information can include an Analytic-ID= VehiclePollution and an Analytic-Result. The Analytic-Result can include a PollutionMap (of the target location, e.g. Madrid), which includes at each geographical coordinate, an intensity level (e.g. from white to black in grey scales) which will be based on: 1) a number of target vehicles (trucks) passing through that geographical coordinate, 2) a time spent by target vehicles (trucks) at that geographical coordinate (e.g. vehicle stopped due to traffic light or traffic jam), having into account vehicle data like Engine ON/OFF (e.g. in case a vehicle turns off the engine while stopped at a traffic light or traffic jam), and 3) a vehicle speed when passing through that geographical coordinate (as emissions depend on the vehicle speed). The Analytic-Result can further include a prediction on vehicle pollution (e.g. based on historical data from previous analytic results) showing the future projection. Optionally, a confidence level (e.g. a percentage from 0% to 100%). The Analytic-Result can further include a list of locations with higher vehicle pollution, a list of vehicles (UE-IDs) or vehicle types which contribute the most to the Vehicle pollution, and statistics (on a per time period, e.g. weekdays vs weekend) on percentage of vehicles of a certain type (e.g. Car, Truck, Bus), brand (e.g. Volvo, Toyota), fuel type (Diesel, Biodiesel, Gasoline, Hybrid, Electric), manufacturing year (e.g. before 2005), etc.

At blocks 590 and 595, the NWDAF 236 can provide the Analytic-Result to the consumer 390. At block 598, the consumer 390 (e.g., an AF as a government or local administration) applies the corresponding actions (e.g., planning relative to vehicle restrictions).

An IoT Analytics based innovation is described below.

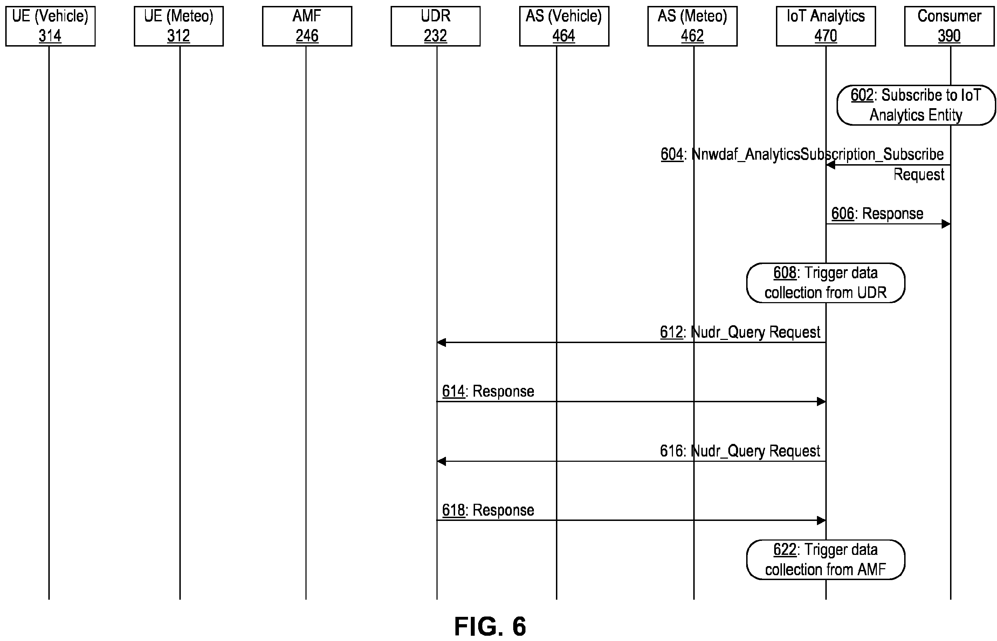

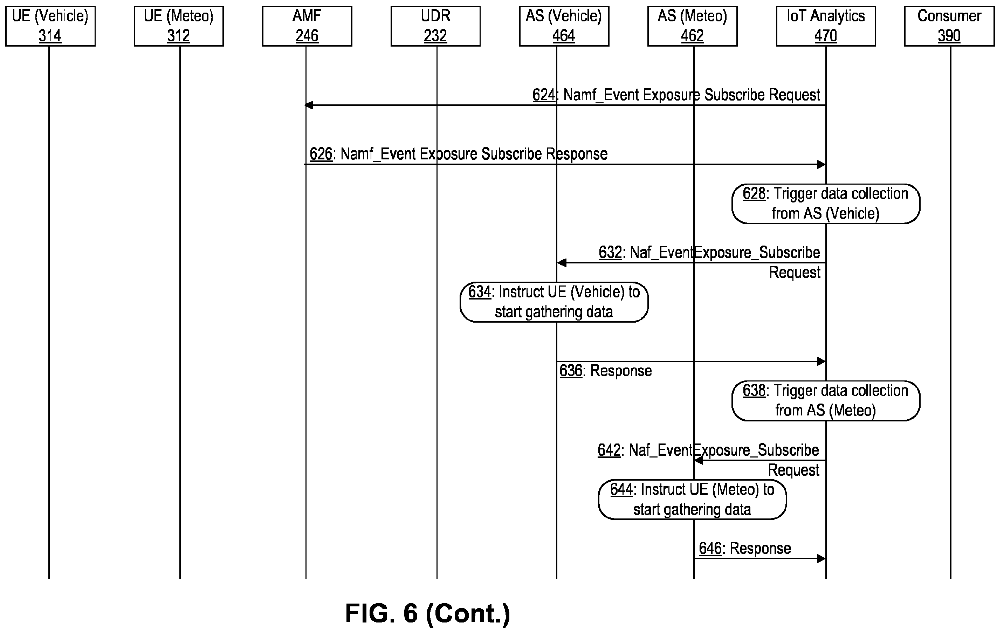

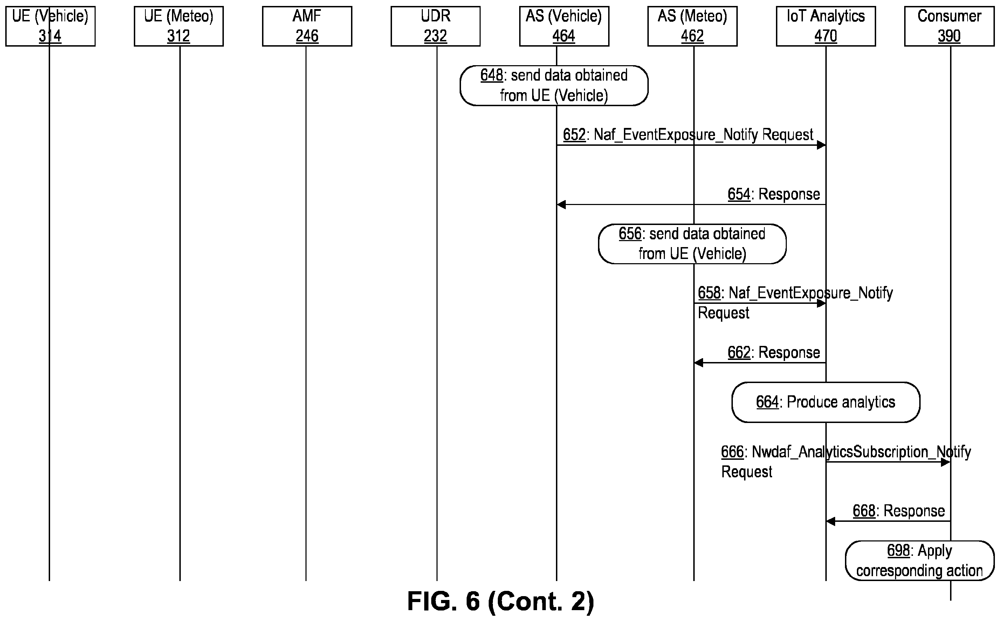

A new IoT Analytics entity (hosted in the IoT Cloud Platform) is the one producing the analytic. FIG. 6 illustrates a sequence diagram describing the proposed mechanism. As a precondition, as part of the subscriber data, the following parameters are stored in the UDR 232: IoT device subscriptions (Vehicles); and IoT device subscriptions (Meteorological stations). The IoT device subscriptions can indicate whether the IoT device is a vehicle. If yes, the IoT device subscription may further indicate a VehicleType (e.g., Car, Truck, Bus, etc), a VehicleBrand (e.g., Volvo, Toyota), a VehicleFuelType (e.g., Diesel, Biodiesel, Gasoline, Hybrid, Electric), and a VehicleManufacturingYear (e.g., 2002). The IoT device subscriptions (can indicate whether the IoT is a meteorological station (with the capability of measuring vehicle pollutants). If yes, the IoT device subscription may further indicate a location of the meteorological station.

FIG. 6 illustrates an example of a signal flow diagram including an IoT Analytics entity 470 that determines vehicle pollution analytics based on information from multiple UEs (e.g., UE 314 associated with a vehicle and UE 312 associated with a meteorological station).

At block 602 and 604, consumer 390 (e.g., an AF associated with a government or a local administration) subscribes to IoT analytics entity 470 (hosted in the IoT Cloud Platform) related to a new analytic (Analytic-ID=VehiclePollution), by transmitting an AnalyticsSubscription_Subscribe Request message to the IoT analytics entity 470. In some examples, the AnalyticsSubscription_Subscribe Request message can include one or more of the following parameters: an analytic ID; an analytic type; a location; a time parameter; a list of UEs; a vehicle type; a vehicle brand; a vehicle fuel type; a vehicle manufacturing year/period; and driving restriction parameters.

The analytic ID (which can be referred to as an Analytic-ID) can be "VehiclePollution."

The analytic type (which can be referred to as Analytic-Type) can be set to statistical, predictive, or both. A statistical analytic type can refer to a request for vehicle pollution analytics based on the analysis of existing data. A predictive analytic type can refer to a request for predictions about the vehicle pollution in the future based on the analysis of existing data and projections into a target date.

The list of locations can include, for example, a single location like a city: Madrid; or a list of locations within a city: Madrid and the different districts within Madrid: Latina, Moratalaz, Centro. The latter requests to calculate the Analytic-ID for each location (e.g. district). In the example shown in the sequence diagram of FIG. 6, Location=Madrid.

The Time-Parameters can indicate to start analytic immediately and to report the analytic result periodically on a per daily basis.

A list of UE can include, for example, a UE-ID to track a certain vehicle or UE-Group-ID to track devices from a certain IoT provider. In the example shown in the sequence diagram of FIG. 6, this parameter is not present.

The VehicleType can include, for example, Any, Car, Truck, Bus, etc. In the example shown in the sequence diagram of FIG. 6, VehicleType=Truck.

The VehicleBrand can include, for example, Any, Volvo, Toyota. In the example shown in the sequence diagram of FIG. 6, this parameter is not present.

The VehicleFuelType can include, for example, Any, Diesel, Biodiesel, Gasoline, Hybrid, Electric. In the example shown in the sequence diagram of FIG. 6, this parameter is not present.

The VehicleManufacturingYearPeriod can include, for example, before 2005. In the example shown in the sequence diagram of FIG. 6, this parameter is not present.

The driving restrictions parameters can include, for example, on a certain date, there will be a driving restriction for vehicles of certain type. In the example shown in the sequence diagram of FIG. 6, this parameter is not present.

In the example shown in the sequence diagram of FIG. 6, AF (local administration) subscribes to truck pollution for the city of Madrid.

At block 606, the IoT Analytics entity 470 answers the request message from block 604 with a successful response (accepting the request).

At block 608, the IoT Analytics entity 470 triggers data collection from the UDR 232/UDM. In the sequence diagram of FIG. 6, data collection from the UDR 232is shown. In this case, IoT Analytics entity 470 requests the UDR 232 for the list of UE-IDs for both the target vehicles 314 and target meteorological stations 312 in the target location. In this example, it is assumed the IoT Analytics entity 470 is trusted, so it does not need to interact with the 5GC entities (e.g., the UDR 232) through NEF.

Not shown in the sequence diagram of FIG. 6, but it is also possible to collect data from a UDM (which stores data for active PDU sessions).

At block 612, the IoT Analytics entity 470 triggers a Nudr_Query request message indicating VehicleType=Truck, and Location=Madrid as input parameters.

At block 614, the UDR 232 finds the UE-IDs matching the input parameters in the request and transmits a Nudr_Query response including the list of UE-IDs (Trucks in Madrid).

At block 616, the IoT Analytics entity 470 triggers a Nudr_Query request message indicating MeteoStations and Location=Madrid as input parameters.

At block 618, the UDR 232 finds the UE-IDs matching the input parameters in the request and transmits a Nudr_Query response including the list of UE-IDs (MeteoStations in Madrid).

At block 622, for each of the UE-IDs in the list retrieved, the IoT Analytics entity 470 discovers the AMF 246 serving the PDU session and, at block 628, triggers data collection from the AMF 246 (for the target vehicles 314). In order to do this, at block 624, the IoT Analytics entity 470 triggers a Namf_Event Exposure Subscribe Request including as parameters: Event-ID=Mobility, Trajectory (e.g. as a single event for simplicity in this example, but in general these are two separate events); UE-ID; and Location=Madrid.

At block 626, if the UE-ID is in the target location, AMF tracks its mobility and trajectory, and reports it in a Namf_Event Exposure Notification Request including as parameters: Event-ID=Mobility,Trajectory; UE-ID; and Mobility Trajectoryinfo.

At blocks 628, for each of the UE-IDs (vehicles) in the list retrieved (the ones inside the target location), IoT Analytics entity 470 triggers data collection from Vehicle AS 464, specifically to retrieve Vehicle data. In order to do this, IoT Analytics entity 470 transmits, at block 632, a Naf_EventExposure_Subscribe request message including the following parameters: Event-ID= VehicleData; and UE-ID.

At block 634 the AS (vehicle) 464 instruct the UE (Vehicle) 314 to start gathering data for Event-ID=VehicleData and, at block 636, answers the request message with a successful response (accepting the request).

At block 638, for each of the UE-IDs (Meteorological Stations) in the list retrieved (the ones inside the target location), IoT Analytics entity 470 triggers data collection from Meteo Station AS 462, specifically to retrieve Meteorological Station data. In order to do this, IoT Analytics entity 470 transmits, at block 642, a Naf_EventExposure_Subscribe request message including the following parameters: Event-ID= MeteoStationData; and UE-ID.

At block 644, the AS (Meteo) 462 instructs the UE (Meteo) 312 to start gathering data for Event-ID=MeteoStationData and, at block 646, answers the request message with a successful response (accepting the request).

At blocks 648 and 652, the AS (Vehicle) 464 sends (e.g. periodically) data for Event-ID=VehicleData, previously obtained from Application client at UE (Vehicle) 314, by triggering a Naf_EventExposure_Notify request including the following parameters: Event-ID= VehicleData; UE-ID; and VehicleData (e.g. GPS positions and timestamps, Vehicle speed, Engine ON/OFF, etc). At block 654, the IoT Analytics entity 470 transmits a response acknowledging the data.

At blocks 656 and 658, the AS (Meteo Station) 462 sends (e.g., periodically) data for Event-ID=MeteoStationData, previously obtained from Application client at UE (Meteo Station) 312, by triggering a Naf_EventExposure_Notify request including the following parameters: Event-ID= MeteoStationData; UE-ID; and MeteostationData, e.g. Geographical coordinates, air pollution data like levels of PM (Particulate matter), VOC (Volatile Organic Compounds), NOx (Nitrogen oxides), CO (Carbon monoxide), SO2 (Sulfur dioxide), Greenhouse gases, etc. At block 662, the IoT Analytics entity 470 transmits a response acknowledging the data.

At blocks 664, the IoT Analytics entity 470 produces analytics based on the data collected from UDR 232/UDM, AMF 246 and AS 462, 464/AF (Vehicle and Meteo Station). Specifically, the NWDAF 236 runs analytic processes (e.g., by using Machine Learning, by identifying patterns or behaviors among the vehicles of certain characteristics and/or by correlating data from the vehicles and the meteorological stations, etc) and generates the information. The information can include an Analytic-ID= VehiclePollution and an Analytic-Result. The Analytic-Result can include a PollutionMap (of the target location, e.g. Madrid), which includes at each geographical coordinate, an intensity level (e.g. from white to black in grey scales) which will be based on: 1) a number of target vehicles (trucks) passing through that geographical coordinate, 2) a time spent by target vehicles (trucks) at that geographical coordinate (e.g. vehicle stopped due to traffic light or traffic jam), having into account vehicle data like Engine ON/OFF (e.g. in case a vehicle turns off the engine while stopped at a traffic light or traffic jam), and 3) a vehicle speed when passing through that geographical coordinate (as emissions depend on the vehicle speed). The Analytic-Result can further include a prediction on vehicle pollution (e.g. based on historical data from previous analytic results) showing the future projection. Optionally, a confidence level (e.g. a percentage from 0% to 100%). The Analytic-Result can further include a list of locations with higher vehicle pollution, a list of vehicles (UE-IDs) or vehicle types which contribute the most to the Vehicle pollution, and statistics (on a per time period, e.g. weekdays vs weekend) on percentage of vehicles of a certain type (e.g. Car, Truck, Bus), brand (e.g. Volvo, Toyota), fuel type (Diesel, Biodiesel, Gasoline, Hybrid, Electric), manufacturing year (e.g. before 2005), etc.

At blocks 666 and 668, the IoT analytics entity 470 can provide the Analytic-Result to the consumer 390. At block 698, the consumer 390 (e.g., an AF as a government or local administration) applies the corresponding actions (e.g., planning relative to vehicle restrictions).

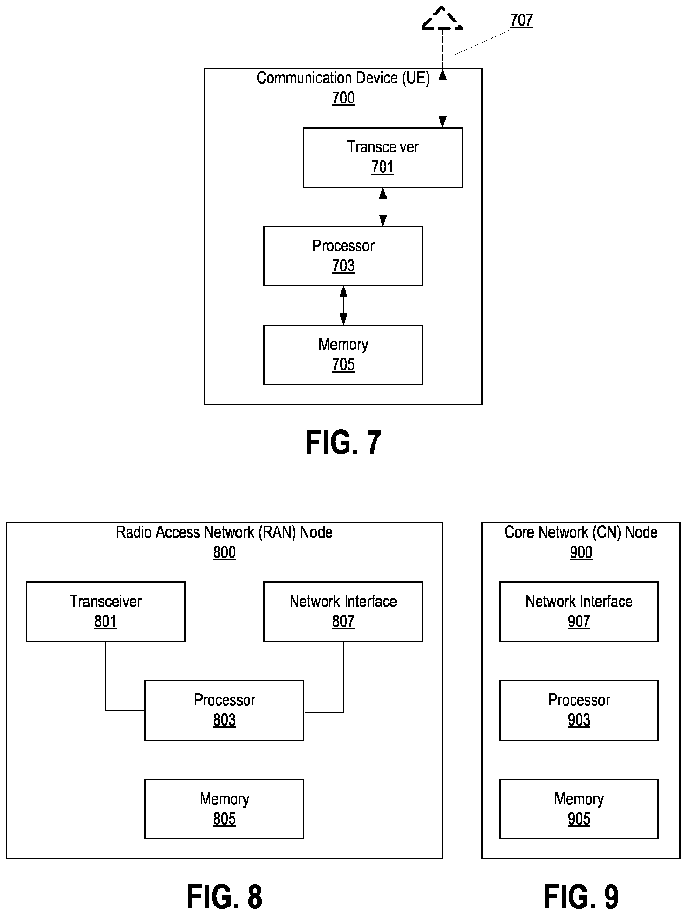

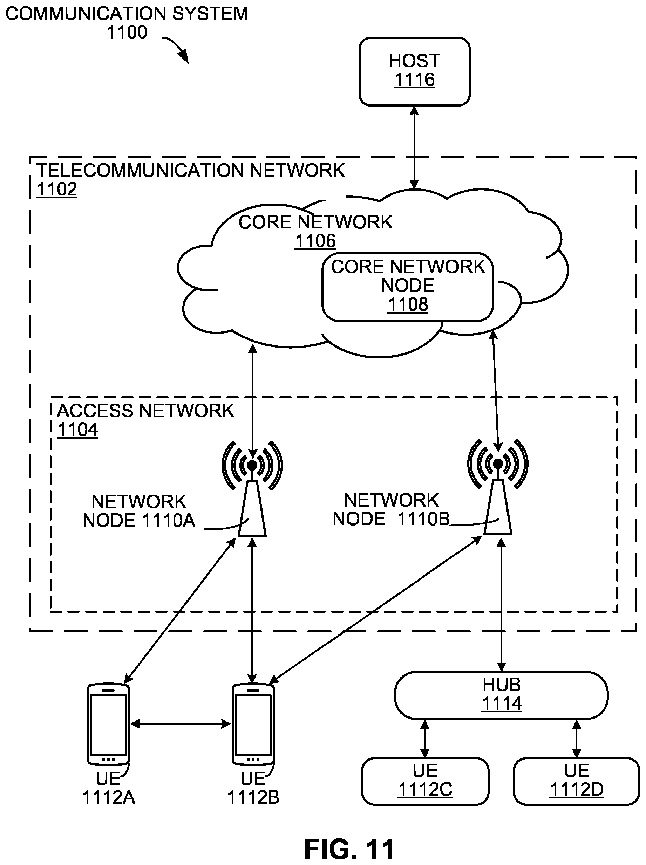

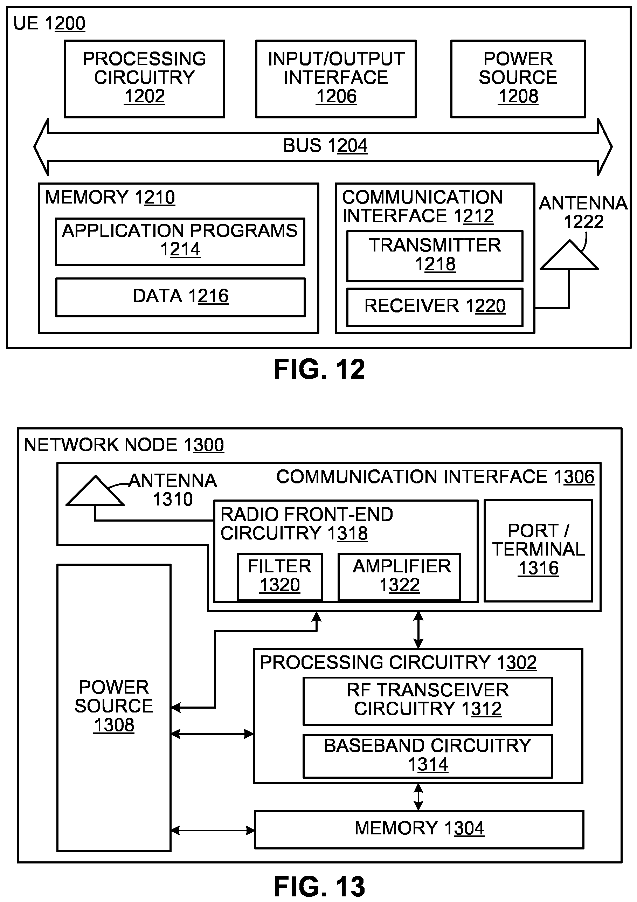

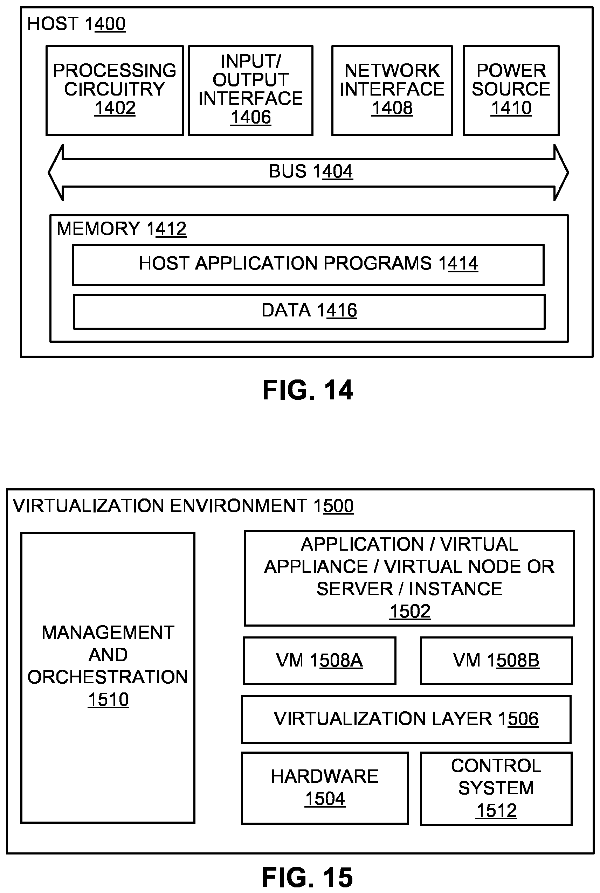

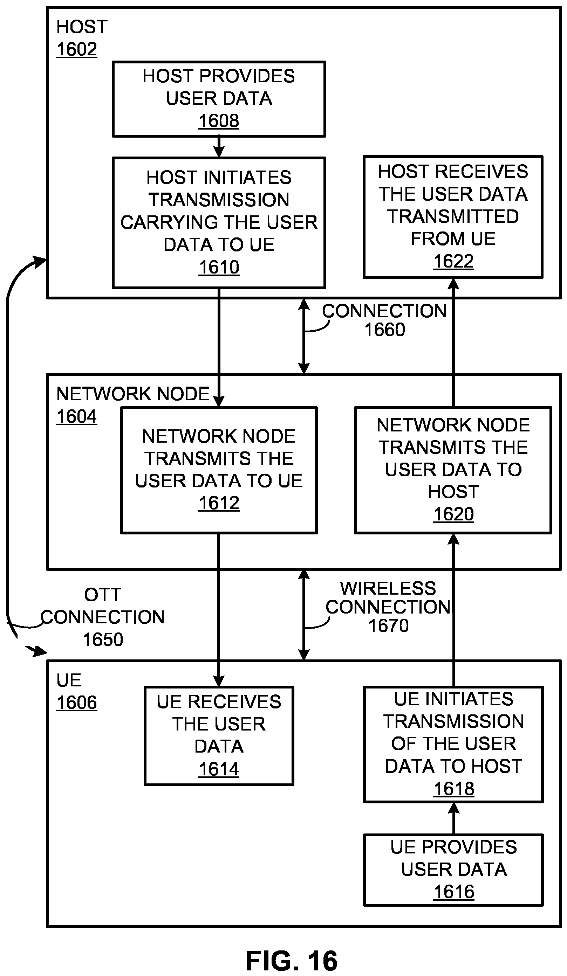

FIG. 7 is a block diagram illustrating elements of a communication device 700 (also referred to as a mobile terminal, a mobile communication terminal, a wireless device, a wireless communication device, a wireless terminal, mobile device, a wireless communication terminal, user equipment ("UE"), a user equipment node/terminal/device, etc.) configured to provide wireless communication according to embodiments of inventive concepts. Communication device 700 may be provided, for example, as discussed below with respect to wireless devices UE 1112A, UE 1112B, and wired or wireless devices UE 1112C, UE 1112D of FIG. 11, UE 1200 of FIG. 12, virtualization hardware 1504 and virtual machines 1508A, 1508B of FIG. 15, and UE 1606 of FIG. 16, all of which should be considered interchangeable in the examples and embodiments described herein and be within the intended scope of this disclosure, unless otherwise noted. As shown, communication device UE may include an antenna 707 (e.g., corresponding to antenna 1222 of FIG. 12), and transceiver circuitry 701 (also referred to as a transceiver, e.g., corresponding to interface 1212 of FIG. 12 having transmitter 1218 and receiver 1220) including a transmitter and a receiver configured to provide uplink and downlink radio communications with a base station(s) (e.g., corresponding to network node 1110A, 1110B of FIG. 11, network node 1300 of FIG. 13, and network node 1604 of FIG. 16 also referred to as a RAN node) of a radio access network. Communication device 700 may also include processing circuitry 703 (also referred to as a processor, e.g., corresponding to processing circuitry 1202 of FIG. 12, and control system 1512 of FIG. 15) coupled to the transceiver circuitry, and memory circuitry 705 (also referred to as memory, e.g., corresponding to memory 1210 of FIG. 11) coupled to the processing circuitry 703. The memory circuitry 705 may include computer readable program code that when executed by the processing circuitry 703 causes the processing circuitry 703 to perform operations according to embodiments disclosed herein. According to other embodiments, processing circuitry 703 may be defined to include memory so that separate memory circuitry is not required. Communication device 700 may also include an interface (such as a user interface) coupled with processing circuitry 703, and/or communication device 700 may be incorporated in a vehicle.

As discussed herein, operations of communication device 700 may be performed by processing circuitry 703 and/or transceiver circuitry 701. For example, processing circuitry 703 may control transceiver circuitry 701 to transmit communications through transceiver circuitry 701 over a radio interface to a radio access network node (also referred to as a base station) and/or to receive communications through transceiver circuitry 701 from a RAN node over a radio interface. Moreover, modules may be stored in memory circuitry 705, and these modules may provide instructions so that when instructions of a module are executed by processing circuitry 703, processing circuitry 703 performs respective operations. According to some embodiments, a communication device 700 and/or an element(s)/function(s) thereof may be embodied as a virtual node/nodes and/or a virtual machine/machines.

FIG. 8 is a block diagram illustrating elements of a radio access network ("RAN") node 800 (also referred to as a network node, base station, eNodeB/eNB, gNodeB/gNB, etc.) of a RAN configured to provide cellular communication according to embodiments of inventive concepts. (RAN node 800 may be provided, for example, as discussed below with respect to network node 1110A, 1110B of FIG. 11, network node 1300 of FIG. 13, hardware 1504 or virtual machine 1508A, 1508B of FIG. 15, and/or base station 1604 of FIG. 16, all of which should be considered interchangeable in the examples and embodiments described herein and be within the intended scope of this disclosure, unless otherwise noted.) As shown, the RAN node may include transceiver circuitry 801 (also referred to as a transceiver, e.g., corresponding to portions of RF transceiver circuitry 1312 and radio front end circuitry 1318 of FIG. 13) including a transmitter and a receiver configured to provide uplink and downlink radio communications with mobile terminals. The RAN node may include network interface circuitry 807 (also referred to as a network interface, e.g., corresponding to portions of communication interface 1306 of FIG. 13) configured to provide communications with other nodes (e.g., with other base stations) of the RAN and/or core network CN. The RAN node 800 may also include processing circuitry 803 (also referred to as a processor, e.g., corresponding to processing circuitry 1302 of FIG. 13) coupled to the transceiver circuitry, and memory circuitry 805 (also referred to as memory, e.g., corresponding to memory 1304 of FIG. 13) coupled to the processing circuitry 803. The memory circuitry 805 may include computer readable program code that when executed by the processing circuitry 803 causes the processing circuitry 803 to perform operations according to embodiments disclosed herein. According to other embodiments, processing circuitry 803 may be defined to include memory so that a separate memory circuitry is not required.

As discussed herein, operations of the RAN node 800 may be performed by processing circuitry 803, network interface 807, and/or transceiver 801. For example, processing circuitry 803 may control transceiver 801 to transmit downlink communications through transceiver 801 over a radio interface to one or more mobile terminals UEs and/or to receive uplink communications through transceiver 801 from one or more mobile terminals UEs over a radio interface. Similarly, processing circuitry 803 may control network interface 807 to transmit communications through network interface 807 to one or more other network nodes and/or to receive communications through network interface from one or more other network nodes. Moreover, modules may be stored in memory 805, and these modules may provide instructions so that when instructions of a module are executed by processing circuitry 803, processing circuitry 803 performs respective operations. According to some embodiments, RAN node 800 and/or an element(s)/function(s) thereof may be embodied as a virtual node/nodes and/or a virtual machine/machines.

According to some other embodiments, a network node may be implemented as a core network CN node without a transceiver. In such embodiments, transmission to a wireless communication device may be initiated by the network node so that transmission to the wireless communication device is provided through a network node including a transceiver (e.g., through a base station or RAN node). According to embodiments where the network node is a RAN node including a transceiver, initiating transmission may include transmitting through the transceiver.

FIG. 9 is a block diagram illustrating elements of a core network ("CN") node 900 (e.g., an SMF (session management function) node, an AMF (access and mobility management function) node, etc.) of a communication network configured to provide cellular communication according to embodiments of inventive concepts. (CN node 900 may be provided, for example, as discussed below with respect to core network node 1108 of FIG. 11, hardware 1504 or virtual machine 1508A, 1508B of FIG. 15, all of which should be considered interchangeable in the examples and embodiments described herein and be within the intended scope of this disclosure, unless otherwise noted). As shown, the CN node 900 may include network interface circuitry 907 configured to provide communications with other nodes of the core network and/or the RAN. The CN node 900 may also include a processing circuitry 903 (also referred to as a processor,) coupled to the network interface circuitry, and memory circuitry 905 (also referred to as memory) coupled to the processing circuitry 903. The memory circuitry 905 may include computer readable program code that when executed by the processing circuitry 903 causes the processing circuitry 903 to perform operations according to embodiments disclosed herein. According to other embodiments, processing circuitry 903 may be defined to include memory so that a separate memory circuitry is not required.

As discussed herein, operations of the CN node 900 may be performed by processing circuitry 903 and/or network interface circuitry 907. For example, processing circuitry 903 may control network interface circuitry 907 to transmit communications through network interface circuitry 907 to one or more other network nodes and/or to receive communications through network interface circuitry from one or more other network nodes. Moreover, modules may be stored in memory 905, and these modules may provide instructions so that when instructions of a module are executed by processing circuitry 903, processing circuitry 903 performs respective operations. According to some embodiments, CN node 900 and/or an element(s)/function(s) thereof may be embodied as a virtual node/nodes and/or a virtual machine/machines.

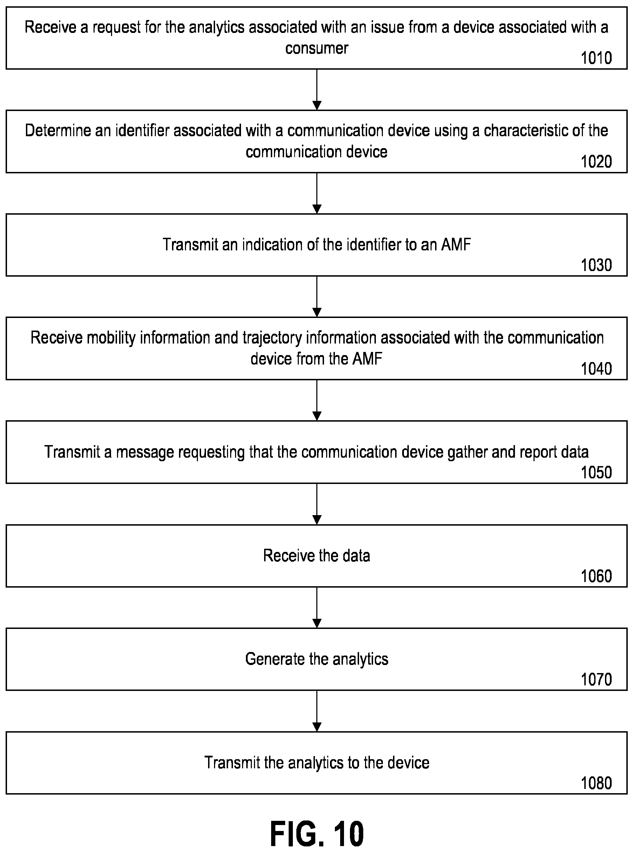

In the description that follows, while the network node may be any of the CN node 900, core network node 1108, hardware 1504, or virtual machine 1508A, 1508B, the CN node 900 shall be used to describe the functionality of the operations of the network node. Operations of the CN node 900 (implemented using the structure of FIG. 9) will now be discussed with reference to the flow chart of FIGS. 10-11 according to some embodiments of inventive concepts. For example, modules may be stored in memory 905 of FIG. 9, and these modules may provide instructions so that when the instructions of a module are executed by respective CN node processing circuitry 903, processing circuitry 903 performs respective operations of the flow chart.

FIG. 10 illustrates operations performed by a network node for determining analytics associated with a communication device. In some embodiments, the network node includes an internet of things, IoT, analytics entity. In additional or alternative embodiments, the network node includes a node configured to perform a network data analytics function, NWDAF.

At block 1010, processing circuitry 903 receives, via network interface 907, a request for the analytics associated with an issue from a device associated with a consumer.

At block 1020, processing circuitry 903 determines an identifier associated with a communication device using a characteristic of the communication device. In some embodiments, determining the identifier associated with the communication device includes transmitting a request for the identifier to a unified data repository, UDR, the request including an indication of the characteristic; and responsive to transmitting the request for the identifier to the UDR, receiving the identifier. In additional or alternative embodiments, the characteristic includes at least one of: whether the communication device is associated with a vehicle, whether the communication device is associated with an information gathering station, a location of the communication device, a vehicle type, a vehicle brand, a vehicle fuel type, and vehicle manufacturing year, a time spent by the vehicle at the location, and a vehicle speed.

At block 1030, processing circuitry 903 transmits, via network interface 907, an indication of the identifier to an AMF.

At block 1040, processing circuitry 903 receives, via network interface 907, mobility information and trajectory information associated with the communication device from the AMF.

At block 1050, processing circuitry 903 transmits, via network interface 907, a message requesting that the communication device gather and report data. In some embodiments, transmitting the message requesting that the communication device gather and report data includes, responsive to determining the identifier, transmitting the message directly to the communication device. In additional or alternative embodiments, transmitting the message requesting that the communication device gather and report data includes responsive to determining the identifier, transmitting the message to the communication device via an application server, AS, associated with the communication device.

In additional or alternative embodiments, transmitting the message requesting that the communication device gather and report data includes transmitting a subscription message requesting that the communication device periodically report the data.

At block 1060, processing circuitry 903 receives, via network interface 907, the data. In some embodiments, receiving the data includes receiving the data directly from the communication device. In additional or alternative embodiments, receiving the data includes receiving the data from the AS

At block 1070, processing circuitry 903 generates the analytics. In some embodiments, generating the analytics includes generating the analytics based on the data, the mobility information, and the trajectory information. In additional or alternative embodiments, generating the analytics includes generating at least one of: statistical analytics; and predictive analytics. In additional or alternative embodiments, generating the analytics includes generating the analytics using a machine learning algorithm.

At block 1080, processing circuitry 903 transmits, via network interface 907, the analytics to the device.

In some embodiments, the issue includes vehicle pollution. The communication device includes a first communication device and a second communication device, the first communication device associated with a vehicle and the second communication device associated with a meteorological station. The data includes first data from the first communication device indicating a location of the vehicle and second data from the second communication device indicating an amount of air pollution at the location.

In additional or alternative embodiments, generating the analytics includes generating statistical information associating the air pollution with the vehicle.

In additional or alternative embodiments, generating the analytics includes generating predictive information estimating air pollution at the location at a future time in response to a potential restriction on vehicles.

Various operations from the flow chart of FIG. 10 may be optional with respect to some embodiments of CN nodes and related methods. For example, operations of blocks 1010, 1030, 1040, 1060, and 1080 of FIG. 10 may be optional.

**FIG. 11** shows an example of a communication system 1100 in accordance with some embodiments.

In the example, the communication system 1100 includes a telecommunication network 1102 that includes an access network 1104, such as a radio access network (RAN), and a core network 1106, which includes one or more core network nodes 1108. The access network 1104 includes one or more access network nodes, such as network nodes 1110a and 1110b (one or more of which may be generally referred to as network nodes 1110), or any other similar 3^{rd} Generation Partnership Project (3GPP) access node or non-3GPP access point. The network nodes 1110 facilitate direct or indirect connection of user equipment (UE), such as by connecting UEs 1112a, 1112b, 1112c, and 1112d (one or more of which may be generally referred to as UEs 1112) to the core network 1106 over one or more wireless connections.

Example wireless communications over a wireless connection include transmitting and/or receiving wireless signals using electromagnetic waves, radio waves, infrared waves, and/or other types of signals suitable for conveying information without the use of wires, cables, or other material conductors. Moreover, in different embodiments, the communication system 1100 may include any number of wired or wireless networks, network nodes, UEs, and/or any other components or systems that may facilitate or participate in the communication of data and/or signals whether via wired or wireless connections. The communication system 1100 may include and/or interface with any type of communication, telecommunication, data, cellular, radio network, and/or other similar type of system.

The UEs 1112 may be any of a wide variety of communication devices, including wireless devices arranged, configured, and/or operable to communicate wirelessly with the network nodes 1110 and other communication devices. Similarly, the network nodes 1110 are arranged, capable, configured, and/or operable to communicate directly or indirectly with the UEs 1112 and/or with other network nodes or equipment in the telecommunication network 1102 to enable and/or provide network access, such as wireless network access, and/or to perform other functions, such as administration in the telecommunication network 1102.

In the context of the analytics functionality described above, the UEs 1112 may particularly correspond to any of the mentioned IoT devices, like IoT Device (Meteo) / UE (Meteo) 312 or IoT Device (Vehicle) / UE (Vehicle) 314 of Figures 4 or 6, or UE 110 of Fig. 5.

In the depicted example, the core network 1106 connects the network nodes 1110 to one or more hosts, such as host 1116. These connections may be direct or indirect via one or more intermediary networks or devices. In other examples, network nodes may be directly coupled to hosts. The core network 1106 includes one more core network nodes (e.g., core network node 1108) that are structured with hardware and software components. Features of these components may be substantially similar to those described with respect to the UEs, network nodes, and/or hosts, such that the descriptions thereof are generally applicable to the corresponding components of the core network node 1108. Example core network nodes include functions of one or more of a Mobile Switching Center (MSC), Mobility Management Entity (MME), Home Subscriber Server (HSS), Access and Mobility Management Function (AMF), Session Management Function (SMF), Authentication Server Function (AUSF), Subscription Identifier De-concealing function (SIDF), Unified Data Management (UDM), Security Edge Protection Proxy (SEPP), Network Exposure Function (NEF), and/or a User Plane Function (UPF). In the context of the analytics functionality described above, core network node 1108 may particularly correspond to one or more of AMF 246, UDR 232, NEF 234 or NWDAF 236 of Figures 4, 5 or 6.

The host 1116 may be under the ownership or control of a service provider other than an operator or provider of the access network 1104 and/or the telecommunication network 1102, and may be operated by the service provider or on behalf of the service provider. The host 1116 may host a variety of applications to provide one or more service. Examples of such applications include data collection services such as retrieving and compiling data on various ambient conditions detected by a plurality of UEs, analytics functionality, functions for controlling or otherwise interacting with remote devices, functions for an alarm and surveillance center, or any other such function performed by a server. In the context of the analytics functionality described above, host 1116 may particularly correspond to the IoT Cloud Platform 330a of Fig. 4 or one of its components, like AS (Meteo) 462, AS (Vehicle) 464 or IoT Analytics Entity 470 of Fig. 4 or Fig. 6. It may also correspond to Data Producer 380 of Fig. 4 or Consumer 390 of Figures 4, 5 or 6.

As a whole, the communication system 1100 of FIG. 11 enables connectivity between the UEs, network nodes, and hosts. In that sense, the communication system may be configured to operate according to predefined rules or procedures, such as specific standards that include, but are not limited to: Global System for Mobile Communications (GSM); Universal Mobile Telecommunications System (UMTS); Long Term Evolution (LTE), and/or other suitable 2G, 3G, 4G, 5G standards, or any applicable future generation standard (e.g., 6G); wireless local area network (WLAN) standards, such as the Institute of Electrical and Electronics Engineers (IEEE) 802.11 standards (WiFi); and/or any other appropriate wireless communication standard, such as the Worldwide Interoperability for Microwave Access (WiMax), Bluetooth, Z-Wave, Near Field Communication (NFC) ZigBee, LiFi, and/or any low-power wide-area network (LPWAN) standards such as LoRa and Sigfox.

In some examples, the telecommunication network 1102 is a cellular network that implements 3GPP standardized features. Accordingly, the telecommunications network 1102 may support network slicing to provide different logical networks to different devices that are connected to the telecommunication network 1102. For example, the telecommunications network 1102 may provide Ultra Reliable Low Latency Communication (URLLC) services to some UEs, while providing Enhanced Mobile Broadband (eMBB) services to other UEs, and/or Massive Machine Type Communication (mMTC)/Massive IoT services to yet further UEs.

In some examples, the UEs 1112 are configured to transmit and/or receive information without direct human interaction. For instance, a UE may be designed to transmit information to the access network 1104 on a predetermined schedule, when triggered by an internal or external event, or in response to requests from the access network 1104. Additionally, a UE may be configured for operating in single- or multi-RAT or multi-standard mode. For example, a UE may operate with any one or combination of Wi-Fi, NR (New Radio) and LTE, i.e. being configured for multi-radio dual connectivity (MR-DC), such as E-UTRAN (Evolved-UMTS Terrestrial Radio Access Network) New Radio - Dual Connectivity (EN-DC).

In the example, the hub 1114 communicates with the access network 1104 to facilitate indirect communication between one or more UEs (e.g., UE 1112c and/or 1112d) and network nodes (e.g., network node 1110b). In some examples, the hub 1114 may be a controller, router, content source and analytics, or any of the other communication devices described herein regarding UEs. For example, the hub 1114 may be a broadband router enabling access to the core network 1106 for the UEs. As another example, the hub 1114 may be a controller that sends commands or instructions to one or more actuators in the UEs. Commands or instructions may be received from the UEs, network nodes 1110, or by executable code, script, process, or other instructions in the hub 1114. As another example, the hub 1114 may be a data collector that acts as temporary storage for UE data and, in some embodiments, may perform analysis or other processing of the data. As another example, the hub 1114 acts as a proxy server or orchestrator for the UEs, in particular in if one or more of the UEs are low energy IoT devices.

The hub 1114 may have a constant/persistent or intermittent connection to the network node 1110b. The hub 1114 may also allow for a different communication scheme and/or schedule between the hub 1114 and UEs (e.g., UE 1112c and/or 1112d), and between the hub 1114 and the core network 1106. In other examples, the hub 1114 is connected to the core network 1106 and/or one or more UEs via a wired connection. Moreover, the hub 1114 may be configured to connect to an M2M service provider over the access network 1104 and/or to another UE over a direct connection. In some scenarios, UEs may establish a wireless connection with the network nodes 1110 while still connected via the hub 1114 via a wired or wireless connection.

**FIG. 12** shows a UE 1200 in accordance with some embodiments. As used herein, a UE refers to a device capable, configured, arranged and/or operable to communicate wirelessly with network nodes and/or other UEs. Examples of a UE include, but are not limited to, a smart phone, mobile phone, cell phone, voice over IP (VoIP) phone, wireless local loop phone, desktop computer, personal digital assistant (PDA), wireless cameras, gaming console or device, music storage device, playback appliance, wearable terminal device, wireless endpoint, mobile station, tablet, laptop, laptop-embedded equipment (LEE), laptop-mounted equipment (LME), smart device, wireless customer-premise equipment (CPE), vehicle-mounted or vehicle embedded/integrated wireless device, etc. Other examples include any UE identified by the 3rd Generation Partnership Project (3GPP), including a narrow band internet of things (NB-IoT) UE, a machine type communication (MTC) UE, and/or an enhanced MTC (eMTC) UE.

A UE may support device-to-device (D2D) communication, for example by implementing a 3GPP standard for sidelink communication, Dedicated Short-Range Communication (DSRC), vehicle-to-vehicle (V2V), vehicle-to-infrastructure (V2I), or vehicle-to-everything (V2X). In other examples, a UE may not necessarily have a user in the sense of a human user who owns and/or operates the relevant device. Instead, a UE may represent a device that is intended for sale to, or operation by, a human user but which may not, or which may not initially, be associated with a specific human user (e.g., a smart sprinkler controller). Alternatively, a UE may represent a device that is not intended for sale to, or operation by, an end user but which may be associated with or operated for the benefit of a user (e.g., a smart power meter).

The UE 1200 includes processing circuitry 1202 that is operatively coupled via a bus 1204 to an input/output interface 1206, a power source 1208, a memory 1210, a communication interface 1212, and/or any other component, or any combination thereof. Certain UEs may utilize all or a subset of the components shown in FIG. 12. The level of integration between the components may vary from one UE to another UE. Further, certain UEs may contain multiple instances of a component, such as multiple processors, memories, transceivers, transmitters, receivers, etc.

The processing circuitry 1202 is configured to process instructions and data and may be configured to implement any sequential state machine operative to execute instructions stored as machine-readable computer programs in the memory 1210. The processing circuitry 1202 may be implemented as one or more hardware-implemented state machines (e.g., in discrete logic, field-programmable gate arrays (FPGAs), application specific integrated circuits (ASICs), etc.); programmable logic together with appropriate firmware; one or more stored computer programs, general-purpose processors, such as a microprocessor or digital signal processor (DSP), together with appropriate software; or any combination of the above. For example, the processing circuitry 1202 may include multiple central processing units (CPUs).

In the example, the input/output interface 1206 may be configured to provide an interface or interfaces to an input device, output device, or one or more input and/or output devices. Examples of an output device include a speaker, a sound card, a video card, a display, a monitor, a printer, an actuator, an emitter, a smartcard, another output device, or any combination thereof. An input device may allow a user to capture information into the UE 1200. Examples of an input device include a touch-sensitive or presence-sensitive display, a camera (e.g., a digital camera, a digital video camera, a web camera, etc.), a microphone, a sensor, a mouse, a trackball, a directional pad, a trackpad, a scroll wheel, a smartcard, and the like. The presence-sensitive display may include a capacitive or resistive touch sensor to sense input from a user. A sensor may be, for instance, an accelerometer, a gyroscope, a tilt sensor, a force sensor, a magnetometer, an optical sensor, a proximity sensor, a biometric sensor, etc., or any combination thereof. An output device may use the same type of interface port as an input device. For example, a Universal Serial Bus (USB) port may be used to provide an input device and an output device.

In some embodiments, the power source 1208 is structured as a battery or battery pack. Other types of power sources, such as an external power source (e.g., an electricity outlet), photovoltaic device, or power cell, may be used. The power source 1208 may further include power circuitry for delivering power from the power source 1208 itself, and/or an external power source, to the various parts of the UE 1200 via input circuitry or an interface such as an electrical power cable. Delivering power may be, for example, for charging of the power source 1208. Power circuitry may perform any formatting, converting, or other modification to the power from the power source 1208 to make the power suitable for the respective components of the UE 1200 to which power is supplied.

The memory 1210 may be or be configured to include memory such as random access memory (RAM), read-only memory (ROM), programmable read-only memory (PROM), erasable programmable read-only memory (EPROM), electrically erasable programmable read-only memory (EEPROM), magnetic disks, optical disks, hard disks, removable cartridges, flash drives, and so forth. In one example, the memory 1210 includes one or more application programs 1214, such as an operating system, web browser application, a widget, gadget engine, or other application, and corresponding data 1216. The memory 1210 may store, for use by the UE 1200, any of a variety of various operating systems or combinations of operating systems.

The memory 1210 may be configured to include a number of physical drive units, such as redundant array of independent disks (RAID), flash memory, USB flash drive, external hard disk drive, thumb drive, pen drive, key drive, high-density digital versatile disc (HD-DVD) optical disc drive, internal hard disk drive, Blu-Ray optical disc drive, holographic digital data storage (HDDS) optical disc drive, external mini-dual in-line memory module (DIMM), synchronous dynamic random access memory (SDRAM), external micro-DIMM SDRAM, smartcard memory such as tamper resistant module in the form of a universal integrated circuit card (UICC) including one or more subscriber identity modules (SIMs), such as a USIM and/or ISIM, other memory, or any combination thereof. The UICC may for example be an embedded UICC (eUICC), integrated UICC (iUICC) or a removable UICC commonly known as 'SIM card.' The memory 1210 may allow the UE 1200 to access instructions, application programs and the like, stored on transitory or non-transitory memory media, to off-load data, or to upload data. An article of manufacture, such as one utilizing a communication system may be tangibly embodied as or in the memory 1210, which may be or comprise a device-readable storage medium.

The processing circuitry 1202 may be configured to communicate with an access network or other network using the communication interface 1212. The communication interface 1212 may comprise one or more communication subsystems and may include or be communicatively coupled to an antenna 1222. The communication interface 1212 may include one or more transceivers used to communicate, such as by communicating with one or more remote transceivers of another device capable of wireless communication (e.g., another UE or a network node in an access network). Each transceiver may include a transmitter 1218 and/or a receiver 1220 appropriate to provide network communications (e.g., optical, electrical, frequency allocations, and so forth). Moreover, the transmitter 1218 and receiver 1220 may be coupled to one or more antennas (e.g., antenna 1222) and may share circuit components, software or firmware, or alternatively be implemented separately.

In the illustrated embodiment, communication functions of the communication interface 1212 may include cellular communication, Wi-Fi communication, LPWAN communication, data communication, voice communication, multimedia communication, short-range communications such as Bluetooth, near-field communication, location-based communication such as the use of the global positioning system (GPS) to determine a location, another like communication function, or any combination thereof. Communications may be implemented in according to one or more communication protocols and/or standards, such as IEEE 802.11, Code Division Multiplexing Access (CDMA), Wideband Code Division Multiple Access (WCDMA), GSM, LTE, New Radio (NR), UMTS, WiMax, Ethernet, transmission control protocol/internet protocol (TCP/IP), synchronous optical networking (SONET), Asynchronous Transfer Mode (ATM), QUIC, Hypertext Transfer Protocol (HTTP), and so forth.

Regardless of the type of sensor, a UE may provide an output of data captured by its sensors, through its communication interface 1212, via a wireless connection to a network node. Data captured by sensors of a UE can be communicated through a wireless connection to a network node via another UE. The output may be periodic (e.g., once every 15 minutes if it reports the sensed temperature), random (e.g., to even out the load from reporting from several sensors), in response to a triggering event (e.g., when moisture is detected an alert is sent), in response to a request (e.g., a user initiated request), or a continuous stream (e.g., a live video feed of a patient).

As another example, a UE comprises an actuator, a motor, or a switch, related to a communication interface configured to receive wireless input from a network node via a wireless connection. In response to the received wireless input the states of the actuator, the motor, or the switch may change. For example, the UE may comprise a motor that adjusts the control surfaces or rotors of a drone in flight according to the received input or to a robotic arm performing a medical procedure according to the received input.

A UE, when in the form of an Internet of Things (IoT) device, may be a device for use in one or more application domains, these domains comprising, but not limited to, city wearable technology, extended industrial application and healthcare. Non-limiting examples of such an IoT device are a device which is or which is embedded in: a connected refrigerator or freezer, a TV, a connected lighting device, an electricity meter, a robot vacuum cleaner, a voice controlled smart speaker, a home security camera, a motion detector, a thermostat, a smoke detector, a door/window sensor, a flood/moisture sensor, an electrical door lock, a connected doorbell, an air conditioning system like a heat pump, an autonomous vehicle, a surveillance system, a weather monitoring device, a vehicle parking monitoring device, an electric vehicle charging station, a smart watch, a fitness tracker, a head-mounted display for Augmented Reality (AR) or Virtual Reality (VR), a wearable for tactile augmentation or sensory enhancement, a water sprinkler, an animal- or item-tracking device, a sensor for monitoring a plant or animal, an industrial robot, an Unmanned Aerial Vehicle (UAV), and any kind of medical device, like a heart rate monitor or a remote controlled surgical robot. A UE in the form of an IoT device comprises circuitry and/or software in dependence of the intended application of the IoT device in addition to other components as described in relation to the UE 1200 shown in FIG. 12.

As yet another specific example, in an IoT scenario, a UE may represent a machine or other device that performs monitoring and/or measurements, and transmits the results of such monitoring and/or measurements to another UE and/or a network node. The UE may in this case be an M2M device, which may in a 3GPP context be referred to as an MTC device. As one particular example, the UE may implement the 3GPP NB-IoT standard. In other scenarios, a UE may represent a vehicle, such as a car, a bus, a truck, a ship and an airplane, or other equipment that is capable of monitoring and/or reporting on its operational status or other functions associated with its operation.

In practice, any number of UEs may be used together with respect to a single use case. For example, a first UE might be or be integrated in a drone and provide the drone's speed information (obtained through a speed sensor) to a second UE that is a remote controller operating the drone. When the user makes changes from the remote controller, the first UE may adjust the throttle on the drone (e.g. by controlling an actuator) to increase or decrease the drone's speed. The first and/or the second UE can also include more than one of the functionalities described above. For example, a UE might comprise the sensor and the actuator, and handle communication of data for both the speed sensor and the actuators.

**FIG. 13** shows a network node 1300 in accordance with some embodiments. As used herein, network node refers to equipment capable, configured, arranged and/or operable to communicate directly or indirectly with a UE and/or with other network nodes or equipment, in a telecommunication network. Examples of network nodes include, but are not limited to, access points (APs) (e.g., radio access points), base stations (BSs) (e.g., radio base stations, Node Bs, evolved Node Bs (eNBs) and NR NodeBs (gNBs)).

Base stations may be categorized based on the amount of coverage they provide (or, stated differently, their transmit power level) and so, depending on the provided amount of coverage, may be referred to as femto base stations, pico base stations, micro base stations, or macro base stations. A base station may be a relay node or a relay donor node controlling a relay. A network node may also include one or more (or all) parts of a distributed radio base station such as centralized digital units and/or remote radio units (RRUs), sometimes referred to as Remote Radio Heads (RRHs). Such remote radio units may or may not be integrated with an antenna as an antenna integrated radio. Parts of a distributed radio base station may also be referred to as nodes in a distributed antenna system (DAS).

Other examples of network nodes include multiple transmission point (multi-TRP) 5G access nodes, multi-standard radio (MSR) equipment such as MSR BSs, network controllers such as radio network controllers (RNCs) or base station controllers (BSCs), base transceiver stations (BTSs), transmission points, transmission nodes, multi-cell/multicast coordination entities (MCEs), Operation and Maintenance (O&M) nodes, Operations Support System (OSS) nodes, Self-Organizing Network (SON) nodes, positioning nodes (e.g., Evolved Serving Mobile Location Centers (E-SMLCs)), and/or Minimization of Drive Tests (MDTs).

The network node 1300 includes a processing circuitry 1302, a memory 1304, a communication interface 1306, and a power source 1308. The network node 1300 may be composed of multiple physically separate components (e.g., a NodeB component and a RNC component, or a BTS component and a BSC component, etc.), which may each have their own respective components. In certain scenarios in which the network node 1300 comprises multiple separate components (e.g., BTS and BSC components), one or more of the separate components may be shared among several network nodes. For example, a single RNC may control multiple NodeBs. In such a scenario, each unique NodeB and RNC pair, may in some instances be considered a single separate network node. In some embodiments, the network node 1300 may be configured to support multiple radio access technologies (RATs). In such embodiments, some components may be duplicated (e.g., separate memory 1304 for different RATs) and some components may be reused (e.g., a same antenna 1310 may be shared by different RATs). The network node 1300 may also include multiple sets of the various illustrated components for different wireless technologies integrated into network node 1300, for example GSM, WCDMA, LTE, NR, WiFi, Zigbee, Z-wave, LoRaWAN, Radio Frequency Identification (RFID) or Bluetooth wireless technologies. These wireless technologies may be integrated into the same or different chip or set of chips and other components within network node 1300.

The processing circuitry 1302 may comprise a combination of one or more of a microprocessor, controller, microcontroller, central processing unit, digital signal processor, application-specific integrated circuit, field programmable gate array, or any other suitable computing device, resource, or combination of hardware, software and/or encoded logic operable to provide, either alone or in conjunction with other network node 1300 components, such as the memory 1304, to provide network node 1300 functionality.

In some embodiments, the processing circuitry 1302 includes a system on a chip (SOC). In some embodiments, the processing circuitry 1302 includes one or more of radio frequency (RF) transceiver circuitry 1312 and baseband processing circuitry 1314. In some embodiments, the radio frequency (RF) transceiver circuitry 1312 and the baseband processing circuitry 1314 may be on separate chips (or sets of chips), boards, or units, such as radio units and digital units. In alternative embodiments, part or all of RF transceiver circuitry 1312 and baseband processing circuitry 1314 may be on the same chip or set of chips, boards, or units.

The memory 1304 may comprise any form of volatile or non-volatile computer-readable memory including, without limitation, persistent storage, solid-state memory, remotely mounted memory, magnetic media, optical media, random access memory (RAM), read-only memory (ROM), mass storage media (for example, a hard disk), removable storage media (for example, a flash drive, a Compact Disk (CD) or a Digital Video Disk (DVD)), and/or any other volatile or non-volatile, non-transitory device-readable and/or computer-executable memory devices that store information, data, and/or instructions that may be used by the processing circuitry 1302. The memory 1304 may store any suitable instructions, data, or information, including a computer program, software, an application including one or more of logic, rules, code, tables, and/or other instructions capable of being executed by the processing circuitry 1302 and utilized by the network node 1300. The memory 1304 may be used to store any calculations made by the processing circuitry 1302 and/or any data received via the communication interface 1306. In some embodiments, the processing circuitry 1302 and memory 1304 is integrated.

The communication interface 1306 is used in wired or wireless communication of signaling and/or data between a network node, access network, and/or UE. As illustrated, the communication interface 1306 comprises port(s)/terminal(s) 1316 to send and receive data, for example to and from a network over a wired connection. The communication interface 1306 also includes radio front-end circuitry 1318 that may be coupled to, or in certain embodiments a part of, the antenna 1310. Radio front-end circuitry 1318 comprises filters 1320 and amplifiers 1322. The radio front-end circuitry 1318 may be connected to an antenna 1310 and processing circuitry 1302. The radio front-end circuitry may be configured to condition signals communicated between antenna 1310 and processing circuitry 1302. The radio front-end circuitry 1318 may receive digital data that is to be sent out to other network nodes or UEs via a wireless connection. The radio front-end circuitry 1318 may convert the digital data into a radio signal having the appropriate channel and bandwidth parameters using a combination of filters 1320 and/or amplifiers 1322. The radio signal may then be transmitted via the antenna 1310. Similarly, when receiving data, the antenna 1310 may collect radio signals which are then converted into digital data by the radio front-end circuitry 1318. The digital data may be passed to the processing circuitry 1302. In other embodiments, the communication interface may comprise different components and/or different combinations of components.

In certain alternative embodiments, the network node 1300 does not include separate radio front-end circuitry 1318, instead, the processing circuitry 1302 includes radio front-end circuitry and is connected to the antenna 1310. Similarly, in some embodiments, all or some of the RF transceiver circuitry 1312 is part of the communication interface 1306. In still other embodiments, the communication interface 1306 includes one or more ports or terminals 1316, the radio front-end circuitry 1318, and the RF transceiver circuitry 1312, as part of a radio unit (not shown), and the communication interface 1306 communicates with the baseband processing circuitry 1314, which is part of a digital unit (not shown).

The antenna 1310 may include one or more antennas, or antenna arrays, configured to send and/or receive wireless signals. The antenna 1310 may be coupled to the radio front-end circuitry 1318 and may be any type of antenna capable of transmitting and receiving data and/or signals wirelessly. In certain embodiments, the antenna 1310 is separate from the network node 1300 and connectable to the network node 1300 through an interface or port.

The antenna 1310, communication interface 1306, and/or the processing circuitry 1302 may be configured to perform any receiving operations and/or certain obtaining operations described herein as being performed by the network node. Any information, data and/or signals may be received from a UE, another network node and/or any other network equipment. Similarly, the antenna 1310, the communication interface 1306, and/or the processing circuitry 1302 may be configured to perform any transmitting operations described herein as being performed by the network node. Any information, data and/or signals may be transmitted to a UE, another network node and/or any other network equipment.

The power source 1308 provides power to the various components of network node 1300 in a form suitable for the respective components (e.g., at a voltage and current level needed for each respective component). The power source 1308 may further comprise, or be coupled to, power management circuitry to supply the components of the network node 1300 with power for performing the functionality described herein. For example, the network node 1300 may be connectable to an external power source (e.g., the power grid, an electricity outlet) via an input circuitry or interface such as an electrical cable, whereby the external power source supplies power to power circuitry of the power source 1308. As a further example, the power source 1308 may comprise a source of power in the form of a battery or battery pack which is connected to, or integrated in, power circuitry. The battery may provide backup power should the external power source fail.

Embodiments of the network node 1300 may include additional components beyond those shown in FIG. 13 for providing certain aspects of the network node's functionality, including any of the functionality described herein and/or any functionality necessary to support the subject matter described herein. For example, the network node 1300 may include user interface equipment to allow input of information into the network node 1300 and to allow output of information from the network node 1300. This may allow a user to perform diagnostic, maintenance, repair, and other administrative functions for the network node 1300.

**FIG. 14** is a block diagram of a host 1400, which may be an embodiment of the host 1116 of FIG. 11, in accordance with various aspects described herein. As used herein, the host 1400 may be or comprise various combinations hardware and/or software, including a standalone server, a blade server, a cloud-implemented server, a distributed server, a virtual machine, container, or processing resources in a server farm. The host 1400 may provide one or more services to one or more UEs.

The host 1400 includes processing circuitry 1402 that is operatively coupled via a bus 1404 to an input/output interface 1406, a network interface 1408, a power source 1410, and a memory 1412. Other components may be included in other embodiments. Features of these components may be substantially similar to those described with respect to the devices of previous figures, such as FIGS. 12-13, such that the descriptions thereof are generally applicable to the corresponding components of host 1400.

The memory 1412 may include one or more computer programs including one or more host application programs 1414 and data 1416, which may include user data, e.g., data generated by a UE for the host 1400 or data generated by the host 1400 for a UE. Embodiments of the host 1400 may utilize only a subset or all of the components shown. The host application programs 1414 may be implemented in a container-based architecture and may provide support for video codecs (e.g., Versatile Video Coding (VVC), High Efficiency Video Coding (HEVC), Advanced Video Coding (AVC), MPEG, VP9) and audio codecs (e.g., FLAC, Advanced Audio Coding (AAC), MPEG, G.711), including transcoding for multiple different classes, types, or implementations of UEs (e.g., handsets, desktop computers, wearable display systems, heads-up display systems). The host application programs 1414 may also provide for user authentication and licensing checks and may periodically report health, routes, and content availability to a central node, such as a device in or on the edge of a core network. Accordingly, the host 1400 may select and/or indicate a different host for over-the-top services for a UE. The host application programs 1414 may support various protocols, such as the HTTP Live Streaming (HLS) protocol, Real-Time Messaging Protocol (RTMP), Real-Time Streaming Protocol (RTSP), Dynamic Adaptive Streaming over HTTP (MPEG-DASH), etc.

**FIG. 15** is a block diagram illustrating a virtualization environment 1500 in which functions implemented by some embodiments may be virtualized. In the present context, virtualizing means creating virtual versions of apparatuses or devices which may include virtualizing hardware platforms, storage devices and networking resources. As used herein, virtualization can be applied to any device described herein, or components thereof, and relates to an implementation in which at least a portion of the functionality is implemented as one or more virtual components. Some or all of the functions described herein may be implemented as virtual components executed by one or more virtual machines (VMs) implemented in one or more virtual environments 1500 hosted by one or more of hardware nodes, such as a hardware computing device that operates as a network node, UE, core network node, or host. Further, in embodiments in which the virtual node does not require radio connectivity (e.g., a core network node or host), then the node may be entirely virtualized.

Applications 1502 (which may alternatively be called software instances, virtual appliances, network functions, virtual nodes, virtual network functions, etc.) are run in the virtualization environment Q400 to implement some of the features, functions, and/or benefits of some of the embodiments disclosed herein.

Hardware 1504 includes processing circuitry, memory that stores software and/or instructions executable by hardware processing circuitry, and/or other hardware devices as described herein, such as a network interface, input/output interface, and so forth. Software may be executed by the processing circuitry to instantiate one or more virtualization layers 1506 (also referred to as hypervisors or virtual machine monitors (VMMs)), provide VMs 1508a and 1508b (one or more of which may be generally referred to as VMs 1508), and/or perform any of the functions, features and/or benefits described in relation with some embodiments described herein. The virtualization layer 1506 may present a virtual operating platform that appears like networking hardware to the VMs 1508.

The VMs 1508 comprise virtual processing, virtual memory, virtual networking or interface and virtual storage, and may be run by a corresponding virtualization layer 1506. Different embodiments of the instance of a virtual appliance 1502 may be implemented on one or more of VMs 1508, and the implementations may be made in different ways. Virtualization of the hardware is in some contexts referred to as network function virtualization (NFV). NFV may be used to consolidate many network equipment types onto industry standard high volume server hardware, physical switches, and physical storage, which can be located in data centers, and customer premise equipment.

In the context of NFV, a VM 1508 may be a software implementation of a physical machine that runs programs as if they were executing on a physical, non-virtualized machine. Each of the VMs 1508, and that part of hardware 1504 that executes that VM, be it hardware dedicated to that VM and/or hardware shared by that VM with others of the VMs, forms separate virtual network elements. Still in the context of NFV, a virtual network function is responsible for handling specific network functions that run in one or more VMs 1508 on top of the hardware 1504 and corresponds to the application 1502.

Hardware 1504 may be implemented in a standalone network node with generic or specific components. Hardware 1504 may implement some functions via virtualization. Alternatively, hardware 1504 may be part of a larger cluster of hardware (e.g. such as in a data center or CPE) where many hardware nodes work together and are managed via management and orchestration 1510, which, among others, oversees lifecycle management of applications 1502. In some embodiments, hardware 1504 is coupled to one or more radio units that each include one or more transmitters and one or more receivers that may be coupled to one or more antennas. Radio units may communicate directly with other hardware nodes via one or more appropriate network interfaces and may be used in combination with the virtual components to provide a virtual node with radio capabilities, such as a radio access node or a base station. In some embodiments, some signaling can be provided with the use of a control system 1512 which may alternatively be used for communication between hardware nodes and radio units.

**FIG. 16** shows a communication diagram of a host 1602 communicating via a network node 1604 with a UE 1606 over a partially wireless connection in accordance with some embodiments. Example implementations, in accordance with various embodiments, of the UE (such as a UE 1112a of FIG. 11 and/or UE 1200 of FIG. 12), network node (such as network node 1110a of FIG. 11 and/or network node 1300 of FIG. 13), and host (such as host 1116 of FIG. 11 and/or host 1400 of FIG. 14) discussed in the preceding paragraphs will now be described with reference to FIG. 16.

Like host 1400, embodiments of host 1602 include hardware, such as a communication interface, processing circuitry, and memory. The host 1602 also includes software, which is stored in or accessible by the host 1602 and executable by the processing circuitry. The software includes a host application that may be operable to provide a service to a remote user, such as the UE 1606 connecting via an over-the-top (OTT) connection 1650 extending between the UE 1606 and host 1602. In providing the service to the remote user, a host application may provide user data which is transmitted using the OTT connection 1650.

The network node 1604 includes hardware enabling it to communicate with the host 1602 and UE 1606. The connection 1660 may be direct or pass through a core network (like core network 1106 of FIG. 11) and/or one or more other intermediate networks, such as one or more public, private, or hosted networks. For example, an intermediate network may be a backbone network or the Internet.

The UE 1606 includes hardware and software, which is stored in or accessible by UE 1606 and executable by the UE's processing circuitry. The software includes a client application, such as a web browser or operator-specific "app" that may be operable to provide a service to a human or non-human user via UE 1606 with the support of the host 1602. In the host 1602, an executing host application may communicate with the executing client application via the OTT connection 1650 terminating at the UE 1606 and host 1602. In providing the service to the user, the UE's client application may receive request data from the host's host application and provide user data in response to the request data. The OTT connection 1650 may transfer both the request data and the user data. The UE's client application may interact with the user to generate the user data that it provides to the host application through the OTT connection 1650.

The OTT connection 1650 may extend via a connection 1660 between the host 1602 and the network node 1604 and via a wireless connection 1670 between the network node 1604 and the UE 1606 to provide the connection between the host 1602 and the UE 1606. The connection 1660 and wireless connection 1670, over which the OTT connection 1650 may be provided, have been drawn abstractly to illustrate the communication between the host 1602 and the UE 1606 via the network node 1604, without explicit reference to any intermediary devices and the precise routing of messages via these devices.

As an example of transmitting data via the OTT connection 1650, in step 1608, the host 1602 provides user data, which may be performed by executing a host application. In some embodiments, the user data is associated with a particular human user interacting with the UE 1606. In other embodiments, the user data is associated with a UE 1606 that shares data with the host 1602 without explicit human interaction. In step 1610, the host 1602 initiates a transmission carrying the user data towards the UE 1606. The host 1602 may initiate the transmission responsive to a request transmitted by the UE 1606. The request may be caused by human interaction with the UE 1606 or by operation of the client application executing on the UE 1606. The transmission may pass via the network node 1604, in accordance with the teachings of the embodiments described throughout this disclosure. Accordingly, in step 1612, the network node 1604 transmits to the UE 1606 the user data that was carried in the transmission that the host 1602 initiated, in accordance with the teachings of the embodiments described throughout this disclosure. In step 1614, the UE 1606 receives the user data carried in the transmission, which may be performed by a client application executed on the UE 1606 associated with the host application executed by the host 1602.

In some examples, the UE 1606 executes a client application which provides user data to the host 1602. The user data may be provided in reaction or response to the data received from the host 1602. Accordingly, in step 1616, the UE 1606 may provide user data, which may be performed by executing the client application. In providing the user data, the client application may further consider user input received from the user via an input/output interface of the UE 1606. Regardless of the specific manner in which the user data was provided, the UE 1606 initiates, in step 1618, transmission of the user data towards the host 1602 via the network node 1604. In step 1620, in accordance with the teachings of the embodiments described throughout this disclosure, the network node 1604 receives user data from the UE 1606 and initiates transmission of the received user data towards the host 1602. In step 1622, the host 1602 receives the user data carried in the transmission initiated by the UE 1606.

One or more of the various embodiments improve the performance of OTT services provided to the UE 1606 using the OTT connection 1650, in which the wireless connection 1670 forms the last segment. More precisely, the teachings of these embodiments may improve the ability of network operators to get real-time data and analytics (e.g., regarding vehicle pollution) and thereby provide benefits such as allowing governments and local administrations to reduce air pollution through more precise regulations.

In an example scenario, factory status information may be collected and analyzed by the host 1602. As another example, the host 1602 may process audio and video data which may have been retrieved from a UE for use in creating maps. As another example, the host 1602 may collect and analyze real-time data to assist in controlling vehicle congestion (e.g., controlling traffic lights). As another example, the host 1602 may store surveillance video uploaded by a UE. As another example, the host 1602 may store or control access to media content such as video, audio, VR or AR which it can broadcast, multicast or unicast to UEs. As other examples, the host 1602 may be used for energy pricing, remote control of non-time critical electrical load to balance power generation needs, location services, presentation services (such as compiling diagrams etc. from data collected from remote devices), or any other function of collecting, retrieving, storing, analyzing and/or transmitting data.

In some examples, a measurement procedure may be provided for the purpose of monitoring data rate, latency and other factors on which the one or more embodiments improve. There may further be an optional network functionality for reconfiguring the OTT connection 1650 between the host 1602 and UE 1606, in response to variations in the measurement results. The measurement procedure and/or the network functionality for reconfiguring the OTT connection may be implemented in software and hardware of the host 1602 and/or UE 1606. In some embodiments, sensors (not shown) may be deployed in or in association with other devices through which the OTT connection 1650 passes; the sensors may participate in the measurement procedure by supplying values of the monitored quantities exemplified above, or supplying values of other physical quantities from which software may compute or estimate the monitored quantities. The reconfiguring of the OTT connection 1650 may include message format, retransmission settings, preferred routing etc.; the reconfiguring need not directly alter the operation of the network node 1604. Such procedures and functionalities may be known and practiced in the art. In certain embodiments, measurements may involve proprietary UE signaling that facilitates measurements of throughput, propagation times, latency and the like, by the host 1602. The measurements may be implemented in that software causes messages to be transmitted, in particular empty or 'dummy' messages, using the OTT connection 1650 while monitoring propagation times, errors, etc.

Although the computing devices described herein (e.g., UEs, network nodes, hosts) may include the illustrated combination of hardware components, other embodiments may comprise computing devices with different combinations of components. It is to be understood that these computing devices may comprise any suitable combination of hardware and/or software needed to perform the tasks, features, functions and methods disclosed herein. Determining, calculating, obtaining or similar operations described herein may be performed by processing circuitry, which may process information by, for example, converting the obtained information into other information, comparing the obtained information or converted information to information stored in the network node, and/or performing one or more operations based on the obtained information or converted information, and as a result of said processing making a determination. Moreover, while components are depicted as single boxes located within a larger box, or nested within multiple boxes, in practice, computing devices may comprise multiple different physical components that make up a single illustrated component, and functionality may be partitioned between separate components. For example, a communication interface may be configured to include any of the components described herein, and/or the functionality of the components may be partitioned between the processing circuitry and the communication interface. In another example, non-computationally intensive functions of any of such components may be implemented in software or firmware and computationally intensive functions may be implemented in hardware.

In certain embodiments, some or all of the functionality described herein may be provided by processing circuitry executing instructions stored on in memory, which in certain embodiments may be a computer program product in the form of a non-transitory computer-readable storage medium. In alternative embodiments, some or all of the functionality may be provided by the processing circuitry without executing instructions stored on a separate or discrete device-readable storage medium, such as in a hard-wired manner. In any of those particular embodiments, whether executing instructions stored on a non-transitory computer-readable storage medium or not, the processing circuitry can be configured to perform the described functionality. The benefits provided by such functionality are not limited to the processing circuitry alone or to other components of the computing device, but are enjoyed by the computing device as a whole, and/or by end users and a wireless network generally.

## Claims

1. A method performed by a network node (900) for determining analytics associated with a communication device, the method comprising:
receiving (1010) a request for the analytics associated with an issue from a device associated with a consumer (390), wherein the issue comprises vehicle pollution wherein the communication device comprises a first communication device and a second communication device, the first communication device (314) associated with a vehicle and the second communication device (312) associated with a meteorological station;
determining (1020) an identifier associated with a communication device using a characteristic of the communication device;
transmitting (1050) a message requesting that the communication device gather and report data, wherein the data comprises a first data from the first communication device (314) indicating a location of the vehicle and a second data from the second communication device (312) indicating an amount of air pollution at the location;
responsive to transmitting the message, receiving (1060) the data;
generating (1070) the analytics based on the data, wherein generating the analytics comprises generating statistical information associating the air pollution with the vehicle; and
responsive to generating the analytics, transmitting (1080) the analytics to the device.

2. The method of Claim 1, wherein determining the identifier associated with the communication device comprises:
transmitting a request for the identifier to a unified data repository, UDR (232), the request including an indication of the characteristic; and
responsive to transmitting the request for the identifier to the UDR (232), receiving the identifier.

3. The method of Claim 1 or 2, wherein the characteristic comprises at least one of: whether the communication device is associated with a vehicle, whether the communication device is associated with an information gathering station, a location of the communication device, a vehicle type, a vehicle brand, a vehicle fuel type, and vehicle manufacturing year, a time spent by the vehicle at the location, and a vehicle speed.

4. The method of any of Claims 1-3, further comprising:
responsive to determining the identifier, transmitting (1030) an indication of the identifier to an access and mobility management function, AMF (246), associated with the communication device; and
responsive to transmitting the indication of the identifier to the AMF (246), receiving (1040) mobility information and/or trajectory information associated with the communication device from the AMF (246),
wherein generating the analytics further comprises generating the analytics based on the data, the mobility information, and/or the trajectory information.

5. The method of any of Claims 1-4, wherein transmitting the message requesting that the communication device gather and report data comprises responsive to determining the identifier, transmitting the message directly to the communication device, and
wherein receiving the data comprises receiving the data directly from the communication device.

6. The method of any of Claims 1-4, wherein transmitting the message requesting that the communication device gather and report data comprises responsive to determining the identifier, transmitting the message to the communication device via an application server, AS (462, 464), associated with the communication device, and
wherein receiving the data comprises receiving the data from the AS (462, 464).

7. The method of any of Claims 5-6, wherein transmitting the message requesting that the communication device gather and report data comprises transmitting a subscription message requesting that the communication device periodically report the data.

8. The method of any of Claims 1-7, wherein generating the analytics comprises generating at least one of: statistical analytics; and predictive analytics, and/or wherein generating the analytics comprises generating the analytics using a machine learning algorithm, and/or wherein the network node comprises an internet of things, IoT, analytics entity (470), and/or wherein the network node comprises a node configured to perform a network data analytics function, NWDAF (236).

9. The method of Claim 1, wherein generating the analytics comprises generating predictive information estimating air pollution at the location at a future time in response to a potential restriction on vehicles.

10. A network node (900) for determining analytics associated with a communication device, the network node comprising:
processing circuitry (903); and
memory (905) coupled to the processing circuitry and having instructions stored therein that are executable by the processing circuitry for causing the network node to perform operations comprising any of the operations of Claims 1-9.

11. A system for determining analytics, comprising
- a network node according to claim 10;
- at least one communication device (312, 314) for which said analytics are determined.

12. The system of claim 11, further comprising a unified data repository, UDR (232), the UDR (232) being configured to
receive a request for an identifier associated with the at least one communication device, the request including an indication of a characteristic of the at least one communication device (312, 314); and
responsive to receiving the request, transmitting the identifier to the network node.

13. The system of claim 11 or 12, further comprising an access and mobility management function, AMF (246), associated with the at least one communication device, the AMF (246) being configured to
receive (1030) an indication of an identifier associated with the at least one communication device (312, 314); and
responsive to receiving the indication of the identifier, transmitting (1040) mobility information and/or trajectory information associated with the communication device to the network node,
wherein the network node is configured to generate the analytics based on the data reported by the at least one communication device (312, 314), the mobility information and/or the trajectory information.

## Patentansprüche

1. Verfahren, das durch einen Netzwerkknoten (900) zum Bestimmen von Analytik, die einer Kommunikationsvorrichtung zugeordnet ist, durchgeführt wird, wobei das Verfahren Folgendes umfasst:
Empfangen (1010) einer Anfrage für die Analytik, die einem Problem zugeordnet ist, von einer Vorrichtung, die einem Konsumenten (390) zugeordnet ist, wobei das Problem Fahrzeugverschmutzung umfasst wobei die Kommunikationsvorrichtung eine erste Kommunikationsvorrichtung und eine zweite Kommunikationsvorrichtung umfasst, die erste Kommunikationsvorrichtung (314) einem Fahrzeug zugeordnet ist und die zweite Kommunikationsvorrichtung (312) einer meteorologischen Station zugeordnet ist;
Bestimmen (1020) einer Kennung, die einer Kommunikationsvorrichtung zugeordnet ist, unter Verwendung einer Eigenschaft der Kommunikationsvorrichtung;
Übertragen (1050) einer Nachricht, die anfragt, dass die Kommunikationsvorrichtung Daten sammelt und meldet, wobei die Daten erste Daten von der ersten Kommunikationsvorrichtung (314), die einen Standort des Fahrzeugs angeben, und zweite Daten von der zweiten Kommunikationsvorrichtung (312), die eine Luftverschmutzungsmenge an dem Standort angeben, umfassen;
als Reaktion auf das Übertragen der Nachricht, Empfangen (1060) der Daten;
Erzeugen (1070) der Analytik basierend auf den Daten, wobei das Erzeugen der Analytik Erzeugen von statistischen Informationen, welche die Luftverschmutzung dem Fahrzeug zuordnen, umfasst; und
als Reaktion auf das Erzeugen der Analytik, Übertragen (1080) der Analytik an die Vorrichtung.

2. Verfahren nach Anspruch 1, wobei das Bestimmen der Kennung, die der Kommunikationsvorrichtung zugeordnet ist, Folgendes umfasst:
Übertragen einer Anfrage für die Kennung an ein einheitliches Datenarchiv, UDR (232), wobei die Anfrage eine Angabe der Eigenschaft beinhaltet; und
als Reaktion auf das Übertragen der Anfrage für die Kennung an das UDR (232), Empfangen der Kennung.

3. Verfahren nach Anspruch 1 oder 2, wobei die Eigenschaft mindestens eines des Folgenden umfasst: ob die Kommunikationsvorrichtung einem Fahrzeug zugeordnet ist, ob die Kommunikationsvorrichtung einer Informationssammelstation zugeordnet ist, einen Standort der Kommunikationsvorrichtung, einen Fahrzeugtyp, eine Fahrzeugmarke, einen Fahrzeugkraftstofftyp, ein Fahrzeugbaujahr, eine durch das Fahrzeug an dem Standort verbrachte Zeit und eine Fahrzeuggeschwindigkeit.

4. Verfahren nach einem der Ansprüche 1-3, ferner umfassend:
als Reaktion auf das Bestimmen der Kennung, Übertragen (1030) einer Angabe der Kennung an eine Zugangs- und Mobilitätsverwaltungsfunktion, AMF (246), die der Kommunikationsvorrichtung zugeordnet ist; und
als Reaktion auf das Übertragen der Angabe der Kennung an die AMF (246), Empfangen (1040) von Mobilitätsinformationen und/oder Trajektorieinformationen, die der Kommunikationsvorrichtung zugeordnet sind, von der AMF (246),
wobei das Erzeugen der Analytik ferner Erzeugen der Analytik basierend auf den Daten, den Mobilitätsinformationen und/oder den Trajektorieinformationen umfasst.

5. Verfahren nach einem der Ansprüche 1-4, wobei das Übertragen der Nachricht, die anfragt, dass die Kommunikationsvorrichtung Daten sammelt und meldet, als Reaktion auf das Bestimmen der Kennung Übertragen der Nachricht direkt an die Kommunikationsvorrichtung umfasst und
wobei das Empfangen der Daten Empfangen der Daten direkt von der Kommunikationsvorrichtung umfasst.

6. Verfahren nach einem der Ansprüche 1-4, wobei das Übertragen der Nachricht, die anfragt, dass die Kommunikationsvorrichtung Daten sammelt und meldet, als Reaktion auf das Bestimmen der Kennung Übertragen der Nachricht an die Kommunikationsvorrichtung über einen Anwendungsserver, AS (462, 464), welcher der Kommunikationsvorrichtung zugeordnet ist, umfasst und
wobei das Empfangen der Daten Empfangen der Daten von dem AS (462, 464) umfasst.

7. Verfahren nach einem der Ansprüche 5-6, wobei das Übertragen der Nachricht, die anfragt, dass die Kommunikationsvorrichtung Daten sammelt und meldet, Übertragen einer Abonnementnachricht umfasst, die anfragt, dass die Kommunikationsvorrichtung periodisch die Daten meldet.

8. Verfahren nach einem der Ansprüche 1-7, wobei das Erzeugen der Analytik Erzeugen mindestens eines des Folgenden umfasst: statistische Analytik; und prädiktive Analytik und/oder wobei das Erzeugen der Analytik Erzeugen der Analytik unter Verwendung eines Algorithmus maschinellen Lernens umfasst und/oder wobei der Netzwerkknoten eine Internet-der-Dinge(IoT)-Analytikentität (470) umfasst und/oder wobei der Netzwerkknoten einen Knoten umfasst, der dazu konfiguriert ist, eine Netzwerkdatenanalytikfunktion, NWDAF (236), durchzuführen.

9. Verfahren nach Anspruch 1, wobei das Erzeugen der Analytik Erzeugen von prädiktiven Informationen umfasst, welche die Luftverschmutzung an dem Standort zu einem zukünftigen Zeitpunkt als Reaktion auf eine potenzielle Einschränkung für Fahrzeuge schätzen.

10. Netzwerkknoten (900) zum Bestimmen von Analytik, die einer Kommunikationsvorrichtung zugeordnet ist, wobei der Netzwerkknoten Folgendes umfasst:
eine Verarbeitungsschaltung (903); und
einen Speicher (905), der an die Verarbeitungsschaltung gekoppelt ist und darauf gespeicherte Anweisungen aufweist, die durch die Verarbeitungsschaltung ausführbar sind, um den Netzwerkknoten zu veranlassen, Vorgänge durchzuführen, die beliebige der Vorgänge der Ansprüche 1-9 umfassen.

11. System zum Bestimmen von Analytik, umfassend:
- einen Netzwerkknoten nach Anspruch 10;
- mindestens eine Kommunikationsvorrichtung (312, 314), für welche die Analytik bestimmt wird.

12. System nach Anspruch 11, ferner umfassend ein einheitliches Datenarchiv, UDR (232), wobei das UDR (232) zu Folgendem konfiguriert ist:
Empfangen einer Anfrage für eine Kennung, die der mindestens einen Kommunikationsvorrichtung zugeordnet ist, wobei die Anfrage eine Angabe einer Eigenschaft der mindestens einen Kommunikationsvorrichtung (312, 314) beinhaltet; und
als Reaktion auf das Empfangen der Anfrage, Übertragen der Kennung an den Netzwerkknoten.

13. System nach Anspruch 11 oder 12, ferner umfassend eine Zugangs- und Mobilitätsverwaltungsfunktion, AMF (246), die der mindestens einen Kommunikationsvorrichtung zugeordnet ist, wobei die AMF (246) zu Folgendem konfiguriert ist:
Empfangen (1030) einer Angabe einer Kennung, die der mindestens einen Kommunikationsvorrichtung (312, 314) zugeordnet ist; und
als Reaktion auf das Empfangen der Angabe der Kennung Übertragen (1040) von Mobilitätsinformationen und/oder Trajektorieinformationen, die der Kommunikationsvorrichtung zugeordnet sind, an den Netzwerkknoten,
wobei der Netzwerkknoten dazu konfiguriert ist, die Analytik basierend auf den Daten, die durch die mindestens eine Kommunikationsvorrichtung (312, 314) gemeldet werden, den Mobilitätsinformationen und/oder den Trajektorieinformationen zu erzeugen.

## Revendications

1. Procédé réalisé par un nœud de réseau (900) pour déterminer des analyses associées à un dispositif de communication, le procédé comprenant :
la réception (1010) d'une demande pour les analyses associées à un problème provenant d'un dispositif associé à un consommateur (390), dans lequel le problème comprend la pollution par les véhicules, dans lequel le dispositif de communication comprend un premier dispositif de communication et un second dispositif de communication, le premier dispositif de communication (314) associé à un véhicule et le second dispositif de communication (312) associé à une station météorologique ;
la détermination (1020) d'un identifiant associé à un dispositif de communication en utilisant une caractéristique du dispositif de communication ;
la transmission (1050) d'un message demandant que le dispositif de communication collecte et rapporte des données, dans lequel les données comprennent une première donnée provenant du premier dispositif de communication (314) indiquant un emplacement du véhicule et une seconde donnée provenant du second dispositif de communication (312) indiquant une quantité de pollution de l'air à l'emplacement ;
en réponse à la transmission du message, la réception (1060) des données ;
la génération (1070) des analyses basées sur les données, dans lequel la génération des analyses comprend la génération d'informations statistiques associant la pollution de l'air avec le véhicule ; et
en réponse à la génération des analyses, la transmission (1080) des analyses au dispositif.

2. Procédé selon la revendication 1, dans lequel la détermination de l'identifiant associé au dispositif de communication comprend :
la transmission d'une demande pour l'identifiant à un référentiel de données unifié, UDR (232), la demande incluant une indication de la caractéristique ; et
en réponse à la transmission de la demande pour l'identifiant à l'UDR (232), la réception de l'identifiant.

3. Procédé selon la revendication 1 ou 2, dans lequel la caractéristique comprend au moins l'une parmi : si le dispositif de communication est associé à un véhicule, si le dispositif de communication est associé à une station de collecte d'informations, un emplacement du dispositif de communication, un type de véhicule, une marque de véhicule, un type de carburant de véhicule, et une année de fabrication de véhicule, un temps passé par le véhicule à l'emplacement, et une vitesse de véhicule.

4. Procédé selon l'une quelconque des revendications 1 à 3, comprenant en outre :
en réponse à la détermination de l'identifiant, la transmission (1030) d'une indication de l'identifiant à une fonction de gestion d'accès et de mobilité, AMF (246), associée au dispositif de communication ; et
en réponse à la transmission de l'indication de l'identifiant à l'AMF (246), la réception (1040) d'informations de mobilité et/ou d'informations de trajectoire associées au dispositif de communication provenant de l'AMF (246),
dans lequel la génération des analyses comprend en outre la génération des analyses basées sur les données, les informations de mobilité, et/ou les informations de trajectoire.

5. Procédé selon l'une quelconque des revendications 1 à 4, dans lequel la transmission du message demandant que le dispositif de communication collecte et rapporte des données comprend, en réponse à la détermination de l'identifiant, la transmission du message directement au dispositif de communication, et
dans lequel la réception des données comprend la réception des données directement provenant du dispositif de communication.

6. Procédé selon l'une quelconque des revendications 1 à 4, dans lequel la transmission du message demandant que le dispositif de communication collecte et rapporte des données comprend, en réponse à la détermination de l'identifiant, la transmission du message au dispositif de communication via un serveur d'application, AS (462, 464), associé au dispositif de communication, et
dans lequel la réception des données comprend la réception des données provenant de l'AS (462, 464).

7. Procédé selon l'une quelconque des revendications 5 à 6, dans lequel la transmission du message demandant que le dispositif de communication collecte et rapporte des données comprend la transmission d'un message d'abonnement demandant que le dispositif de communication rapporte périodiquement les données.

8. Procédé selon l'une quelconque des revendications 1 à 7, dans lequel la génération des analyses comprend la génération d'au moins l'une parmi : des analyses statistiques ; et des analyses prédictives, et/ou dans lequel la génération des analyses comprend la génération des analyses en utilisant un algorithme d'apprentissage automatique, et/ou dans lequel le nœud de réseau comprend une entité d'analyse de l'internet des objets, IoT (470), et/ou dans lequel le nœud de réseau comprend un nœud configuré pour réaliser une fonction d'analyse de données de réseau, NWDAF (236).

9. Procédé selon la revendication 1, dans lequel la génération des analyses comprend la génération d'informations prédictives estimant la pollution de l'air à l'emplacement à un moment futur en réponse à une restriction potentielle sur les véhicules.

10. Nœud de réseau (900) pour déterminer des analyses associées à un dispositif de communication, le nœud de réseau comprenant :
des circuits de traitement (903) ; et
une mémoire (905) couplée aux circuits de traitement et ayant des instructions stockées dans celle-ci qui sont exécutables par les circuits de traitement pour amener le nœud de réseau à réaliser des opérations comprenant l'une quelconque des opérations des revendications 1 à 9.

11. Système pour déterminer des analyses, comprenant
- un nœud de réseau selon la revendication 10 ;
- au moins un dispositif de communication (312, 314) pour lequel lesdites analyses sont déterminées.

12. Système selon la revendication 11, comprenant en outre un référentiel de données unifié, UDR (232), l'UDR (232) étant configuré pour
recevoir une demande pour un identifiant associé à l'au moins un dispositif de communication, la demande incluant une indication d'une caractéristique de l'au moins un dispositif de communication (312, 314) ; et
en réponse à la réception de la demande, transmettre l'identifiant au nœud de réseau.

13. Système selon la revendication 11 ou 12, comprenant en outre une fonction de gestion d'accès et de mobilité, AMF (246), associée à l'au moins un dispositif de communication, l'AMF (246) étant configurée pour
recevoir (1030) une indication d'un identifiant associé à l'au moins un dispositif de communication (312, 314) ; et
en réponse à la réception de l'indication de l'identifiant, transmettre (1040) des informations de mobilité et/ou des informations de trajectoire associées au dispositif de communication au nœud de réseau,
dans lequel le nœud de réseau est configuré pour générer les analyses basées sur les données rapportées par l'au moins un dispositif de communication (312, 314), les informations de mobilité et/ou les informations de trajectoire.
